# EUROPÄISCHE PATENTSCHRIFT

(11) **EP 2 515 973 B1**
(45) Veröffentlichungstag und Bekanntmachung des Hinweises auf die Patenterteilung: **21.02.2018**
(21) Anmeldenummer: 09796743.4
(22) Anmeldetag: 23.12.2009
(51) Int. Cl.: A61M 5/315, A61M 5/32, A61M 5/31

(54) **INJEKTIONSVORRICHTUNG MIT EINER NADELSCHUTZHÜLSE**
INJECTION DEVICE COMPRISING A NEEDLE PROTECTION SLEEVE
DISPOSITIF D'INJECTION COMPORTANT UNE GAINE DE PROTECTION D'AIGUILLE

(43) Veröffentlichungstag der Anmeldung: 31.10.2012
(73) Patentinhaber: Tecpharma Licensing AG, 3401 Burgdorf (CH)
(72) Erfinder: KIRCHHOFER, Fritz, CH-3454 Sumiswald (CH)
(86) Internationale Anmeldenummer: PCT/EP2009/067885
(87) Internationale Veröffentlichungsnummer: WO 2011/076280

(56) Entgegenhaltungen:
- WO-A1-2008/025179
- WO-A1-2008/077706
- WO-A2-2008/050158
- FR-A1- 2 884 723
- US-A- 5 681 291
- US-A1- 2007 118 081

## Beschreibung

Die Erfindung betrifft eine Vorrichtung mit einer Nadelschutzhülse zum Schutz vor Verletzungen an einer Injektionsnadel, mit der ein flüssiges Produkt, vorzugsweise ein Medikament in einen Patienten injizierbar ist. Die Erfindung betrifft insbesondere eine Verabreichungsvorrichtung mit einer Nadelschutzhülse, wobei die Vorrichtung besonders geeignet ist, ein Medikament auf der Basis von FSH oder einer FSH-Variante auszuschütten. Ferner kann die Vorrichtung geeignet sein zur Ausschüttung von Neuroleptika, gefäßerweiternden Mitteln, Blutprodukten, Medikamenten zur Behandlung von rheumatischen Erkrankungen, Onkologika oder Medikamenten zur Behandlung von Infektionskrankheiten. Als flüssige Medikamente werden im Sinne der Erfindung nicht nur Flüssigkeiten im engeren Sinn angesehen, sondern auch pasten- und gelartige Medikamente, solange sich derartige Medikamente nur vergleichbar einer Flüssigkeit fördern lassen. Die Verabreichungsvorrichtung ist vorzugsweise ein Injektionsgerät für die Verabreichung mittels einer infundierenden Injektionsnadel, bevorzugt für eine subkutane Injektion, kann grundsätzlich aber auch ein Injektionsgerät für eine nadellose Verabreichung oder beispielsweise auch ein Inhalationsgerät sein. Injektionsvorrichtungen in Form so genannter Injektionspens sind besonders bevorzugte Ausführungsbeispiele.

Aus der EP 0 956 875 A2 ist eine Vorrichtung zum dosierten Verabreichen eines injizierbaren Produkts bekannt. Bei dieser Vorrichtung wird zum Abziehen der Nadelschutzkappe ein Abstreifer gegen die Kraft einer Feder in eine Nadelschutzhülse geschoben, wobei ein an dem Abstreifer befestigtes Eingriffselement in die Nadelschutzkappe eingreift und diese Kappe bei einer Bewegung des Abstreifers aus der Nadelschutzhülse in distale Richtung mitnimmt und von der Nadel entfernt.

WO 2008/077706 A1 offenbart, in den Ausführungsbeispielen der Figuren 14-15c, eine Vorrichtung zum Schutz vor Verletzungen an einer Injektionsnadel mit allen Merkmalen des Oberbegriffs des Anspruchs 1. Der Erfindung liegt die Aufgabe zu Grunde, eine Vorrichtung zum Schutz vor Verletzungen an einer Nadel zu schaffen, die einfach und kostengünstig aufgebaut und vom Verwender intuitiv bedienbar ist.

Die Aufgabe wird gelöst mit dem Gegenstand des unabhängigen Anspruchs 1. Vorteilhafte Weiterbildungen ergeben sich aus den abhängigen Ansprüchen, der Beschreibung und den Figuren.

Die Erfindung geht aus von einer Vorrichtung, insbesondere Injektionsvorrichtung, zum Schutz vor Verletzungen an einer Injektionsnadel. Die Vorrichtung umfasst ein Gehäuse, eine Nadel, mit der ein flüssiges Produkt injizierbar ist, und eine Nadelschutzhülse. Die Nadelschutzhülse kann aus einer ausgefahrenen Position, insbesondere aus einer Nadelschutzposition entgegen der Kraft einer Feder in eine eingefahrene Position, insbesondere einer Position, in der die Nadel für ein Einstechen freigegeben ist, oder/und aus einer eingefahrenen Position durch die Kraft der Feder in eine ausgefahrene Position bewegbar sein. Insbesondere kann die Feder eine in ihr gespeicherte Energie als in distale Richtung wirkende Kraft auf die Nadelschutzhülse ausüben, insbesondere in der Ausgangsposition und/oder der eingefahrenen Position der Nadelschutzhülse und/oder der oder den ausgefahrenen Position(en). Erfindungsgemäß ist die Feder an der Nadelschutzhülse einteilig gebildet. Beim Zusammenbau der Vorrichtung müssen bei einer einzelnen Feder und einer davon separaten Nadelschutzhülse zwei einzelne Teile in das Gehäuse integriert werden. Stattdessen ist es mit der Erfindung möglich, die Nadelschutzhülse einschließlich Feder als ein einziges Teil in das Gehäuse zu integrieren. Damit können einerseits die Kosten beim Zusammenbau der Vorrichtung und andererseits die Kosten bei der Herstellung des einzelnen Teils gesenkt werden. Statt zwei Werkzeugen wird nur ein Herstellungswerkzeug, wie z. B. eine Spritzgussform für den Kunststoff, aus dem die Nadelschutzhülse einschließlich Feder gebildet ist, benötigt.

In einer Ausführung kann die Feder eine Druckfeder, wie z. B. eine Wendelfeder sein, die am proximalen Ende der Nadelschutzhülse gebildet ist und sich in proximale Richtung erstreckt. Die Feder kann auch mehrere zusammengesetzte, insbesondere symmetrisch zusammengesetzte Biegebalken umfassen. Erfindungsgemäß ist die Feder ein Federarm, der auf Durchbiegung oder mit einem Biegemoment belastbar ist und dadurch federnd wirkt. Die Biegung des Federarms findet insbesondere quer zur Federarmlängsachse statt. Z. B. kann der Federarm im Wesentlichen starr sein und im Wesentlichen nur an einer Stelle, beispielsweise an der Stelle elastisch verformbar sein, an der er an die Nadelschutzhülse mündet. In einem alternativen, bevorzugten Beispiel ist der Federarm an sich, insbesondere über seine gesamte Länge elastisch verformbar, d. h. federnd. Der Federarm weist erfindungsgemäß ein Eingriffsmittel auf, das an eine quer zur Nadellängsachse geneigte Führungsfläche einer Ausnehmung angreift. Bevorzugt greift das Eingriffsmittel von innen her, alternativ von außen in die Ausnehmung ein. Die Führungsfläche kann eine Flanke einer von dem Gehäuse gebildeten Ausnehmung, insbesondere Nut oder Tasche sein, welche eine Kulisse bildet. Die Nut oder Tasche kann durchgehend sein oder einen Nut- oder Taschengrund aufweisen. Bevorzugt ist die Ausnehmung zur Innenseite oder Außenseite des Gehäuses hin offen. Die Nut oder Tasche kann von außerhalb der Vorrichtung sichtbar sein oder nicht. Das Eingriffsmittel kann sich relativ zu der Führungsfläche bewegen, insbesondere an der Führungsfläche entlang, vorzugsweise parallel dazu bewegen. Das Eingriffsmittel drückt bevorzugt gegen die Führungsfläche, wobei die durch die Feder auf die Nadelschutzhülse aufgebrachte Kraft ein von der Neigung der Führungsfläche abhängiger Kraftanteil ist. Insbesondere gilt, je stärker die Führungsfläche in Bezug auf die Nadellängsachse geneigt ist, desto größer der in Verschieberichtung der Nadelschutzhülse wirkende Kraftanteil.

Insbesondere ist die von der Feder unmittelbar ausgeübte Kraft über den Eingriff des Eingriffsmittels in die Führungsfläche in eine auf die Nadelschutzhülse wirkende und insbesondere in Verschieberichtung der Nadelschutzhülse weisende Axialkraft umwandelbar. Bevorzugt kann der Federarm von dem proximalen Ende der Nadelschutzhülse in proximale Richtung abragen. Der Arm kann sich entlang der Längsachse der Vorrichtung oder der Nadelschutzhülse erstrecken wie z. B. parallel in zumindest einem entspannten Zustand des Federarms. Besonders bevorzugt ist das Eingriffsmittel quer zur Bewegungsrichtung der Nadelschutzhülse federnd bewegbar. Das Eingriffsmittel ist bevorzugt in Bewegungsrichtung der Nadelschutzhülse im Wesentlichen axial fest im Bezug auf die Nadelschutzhülse.

Die Vorrichtung kann Teil einer Injektionsvorrichtung sein, das heißt die Erfindung kann von einer Injektionsvorrichtung ausgehen, in die die Vorrichtung zum Schutz vor Verletzungen an einer Injektionsnadel integriert ist. Das Gehäuse der Injektionsvorrichtung kann dem Gehäuse der Vorrichtung entsprechen. Alternativ kann die Vorrichtung eine separate Einheit sein, die an einer Injektionsvorrichtung, wie zum Beispiel einem Autoinjektor oder einer anderen Injektionsvorrichtung befestigbar ist. Hierzu kann die Vorrichtung z. B. ein Gehäuse aufweisen, das mit dem Gehäuse der Injektionsvorrichtung verbindbar ist.

Die Vorrichtung kann in bevorzugten Ausführungen ein Gehäuse und eine Nadel umfassen, die bevorzugt in Längsrichtung des bevorzugt hülsenförmigen Gehäuses angeordnet ist. Die Nadel kann eine an einem Produktbehältnis zum Beispiel mit einer Luer-Lock-Verbindung befestigbare Nadel, insbesondere eine Kanüle sein. Die Kanüle kann einen Nadelträger aufweisen, der mit der Karpule oder einem Karpulenhalter, der die Karpule aufnimmt, verbindbar ist. Die Karpule kann ein Septum aufweisen, das von der Nadel der Kanüle durchstochen werden kann, wenn die Kanüle an der Karpule oder dem Karpulenhalter befestigt ist. Am proximalen Ende ist das Medikament in der Karpule mit einem elastischen Kolben, der dichtend und verschiebbar an der Innenwandung der Karpule anliegt verschlossen.

Das Produktbehältnis kann in einer bevorzugten Alternative eine Spritze sein, an deren vorderen Ende die Nadel fest angeordnet ist. Es ist aber auch eine Spritze denkbar, an der die Nadel, z.B. als Kanüle, befestigbar ist. Eine Spritze weist in der Regel kein Septum, aber einen Kolben wie die Karpule auf. Die Spritze kann an ihrem hinteren, d.h. proximalen Ende einen nach außen abragenden Flansch aufweisen, der auch als Fingerflansch bezeichnet wird, weil er bei einer manuellen Verwendung der Spritze gegen Zeige- und Mittelfinger anschlägt, während mit dem Daumen ein Druck z. B. über eine Kolbenstange auf den Kolben ausgeübt wird.

Die Nadel zum Injizieren des bevorzugt flüssigen Produkts kann optional durch eine Nadelschutzkappe abgedeckt sein. Die Nadelschutzkappe dient vorwiegend dem Zweck, die Nadel steril zu halten und ggf. auch vor mechanischer Beschädigung zu schützen. Die Nadelschutzkappe ist bevorzugt form- und/oder reibschlüssig mit dem Nadelträger, der separat an dem Produktbehältnis befestigbar ist oder Teil des bevorzugt als Spritze ausgebildeten Produktbehältnisses ist, befestigt oder befestigbar. Der Nadelträger kann auch als Befestigungsabschnitt bezeichnet werden. Die Nadelschutzkappe ist bevorzugt in einem Ausgangszustand der Vorrichtung an der Nadel angeordnet. Die Nadelschutzkappe kann beispielsweise aus einem elastischen Material, wie z. B. Kautschuk oder Gummi sein oder zumindest ein solches Material aufweisen. Beispielsweise kann eine Nadelschutzhülse in Verbundbauweise vorteilhaft sein, bei der die aus dem elastischen Material gebildete Kappe von einem in Bezug auf die Kappe festen Kunststoff umgeben wird. Die Nadelschutzkappe wird dadurch stabiler und kann die Nadel noch besser vor mechanischer Beschädigung schützen. Die Nadelschutzkappe wird vor der Injektion von der Nadel entfernt.

Allgemein dient die Nadelschutzhülse zumindest nach bevorzugt auch vor einer Injektion dazu, den Verwender vor unbeabsichtigten Nadelstichen zu schützen. Da ohne Nadelschutzhülse die Nadelspitze nach dem Herausziehen der Nadel aus dem Körpergewebe freiliegen würde, bestünde Verletzungsgefahr oder Injektionsgefahr für Dritte. Um diese Gefahren zu verringern, ist die Nadelschutzhülse über der über das distale Ende des Gehäuses hervorstehenden Nadel angeordnet. Insbesondere umgibt die Nadelschutzhülse die Nadel seitlich. Insbesondere kann die Nadelschutzhülse distal über das distale Ende der Nadel, d. h. die Nadelspitze überstehen. Die Nadelschutzhülse kann relativ zum Gehäuse mit einer Bewegung in distale Richtung, d.h. in die Richtung, in die die Nadelspitze weist, bewegt, insbesondere ausgefahren werden. In der ausgefahrenen Position ist die Nadelschutzhülse bevorzugt über der Nadel und der Nadelspitze angeordnet.

Die Nadelschutzhülse kann zwischen einer ausgefahrenen Position, die in bevorzugten Ausführungen die Ausgangsposition der Nadelschutzhülse ist, die sie im Ausgangs- oder Auslieferungszustand der Vorrichtung einnimmt, relativ zu dem Gehäuse in eine eingefahrene Position bewegbar sein, in der die Nadel über das distale Ende der Nadelschutzhülse hervorsteht, wie beispielsweise bei einem Injektionspen für eine manuelle Produktausschüttung, oder zumindest in eine hervorstehende Position bewegt werden kann, wie beispielsweise bei einem Autoinjektor, bei dem die Nadel automatisch eingestochen und insbesondere das Produkt automatisch ausgeschüttet wird. Bevorzugt ist die Nadelschutzkappe durch Andrücken ihres distalen Endes an eine Injektionsstelle in die eingefahrene Position bewegbar, wodurch die Nadel in das Körpergewebe einstechbar ist. Durch die Bewegung der Nadelschutzkappe in die eingefahrene Position kann die relativ zum Gehäuse feststehende Nadel aus der Nadelschutzhülse hervortreten oder kann ein Vortriebsmechanismus, z.B. mit einer Vortriebsfeder, ausgelöst werden, der die Nadel relativ zum Gehäuse in distale Richtung verschiebt, wodurch die Nadel aus der Nadelschutzhülse hervortritt. In der zuletzt genannten Alternative kann die Nadelschutzhülse auch als Auslöseelement dienen.

Die Nadelschutzhülse ist bevorzugt aus ihrer eingefahrenen Position in eine ausgefahrene Position bewegbar, indem die Nadelschutzhülse in distale Richtung bewegt wird. Die ausgefahrenen Positionen vor und nach der Bewegung der Nadelschutzhülse in die eingefahrene Position können gleich oder unterschiedlich sein. Insbesondere kann die Nadelschutzhülse in unterschiedlichen ausgefahrenen Positionen unterschiedlich weit von dem distalen Ende des Gehäuses abstehen. Allgemein bevorzugt wird in den ausgefahrenen Positionen die Nadelspitze von der Nadelschutzhülse abgedeckt. Insbesondere ist die Nadelschutzhülse in ihren relativ zum Gehäuse ausgefahrenen Positionen an der Vorrichtung insbesondere an dem Gehäuse der Vorrichtung befestigt.

Bevorzugt ist die Nadelschutzhülse am Ende der Bewegung aus der ausgefahrenen Position in die eingefahrene Position zurück in eine ausgefahrene Position an einer Bewegung in distale und proximale Richtung gesperrt. Vorteilhaft ist die Nadelschutzhülse nach der Verwendung der Vorrichtung axial fest insbesondere mit und relativ zu dem Gehäuse verriegelt. Dadurch kann die Nadelschutzhülse nicht mehr in das Gehäuse zurückgeschoben werden, wodurch die Nadel nicht mehr aus dem distalen Ende der Nadelschutzhülse hervortreten kann und wodurch die von der Nadel ausgehende Verletzungsgefahr verringert wird.

Zum einfacheren Verständnis wird die ausgefahrene Position, in der die Nadelschutzhülse aus der Ausgangsposition in distale Richtung bewegt oder in der Ausgangsposition ist, und insbesondere in einen Eingriff mit dem in proximale Richtung wirkenden Anschlag ist, als erste ausgefahrene Position bezeichnet. Die Position der Nadelschutzhülse, in der sie aus der eingefahrenen Position in die ausgefahrene Position bewegt und insbesondere axial fest verriegelt ist, wird als zweite ausgefahrene Position bezeichnet. Erste und zweite ausgefahrene Position können hinsichtlich ihrer Position relativ zu dem Gehäuse gleich sein, müssen es aber nicht. Zum Beispiel kann die Nadelschutzhülse in der ersten ausgefahrenen Position weiter von dem Gehäuse abstehen als in der zweiten ausgefahrenen Position, oder umgekehrt.

Die Nadelschutzhülse kann gegen die Kraft des Federelements verschiebbar sein, d. h., dass durch die Verschiebung der Nadelschutzhülse in proximale Richtung, insbesondere aus der ersten ausgefahrenen Position in die eingefahrene Position, das Federelement gespannt wird.

Die Nadelschutzhülse weist, zusätzlich zu dem erfindungsgemäßen Eingriffsmittel, bevorzugt ein Eingriffsglied auf, das z.B. in das Gehäuse eingreift. Das Eingriffsglied, das bevorzugt nockenförmig ist, kann relativ zur Nadelschutzhülse federnd gelagert sein. Insbesondere kann die federnde Lagerung eine Bewegung des Eingriffsglieds quer zur Längsachse der Vorrichtung oder der Nadelschutzhülse zulassen, wobei das Eingriffsglied bevorzugt in der Längsrichtung der Vorrichtung oder der Nadelschutzhülse, insbesondere relativ zur Nadelschutzhülse keine oder nur eine vernachlässigbare Bewegung ausführen kann. Dies kann insbesondere durch einen Arm erreicht werden, der mit einem Ende insbesondere einteilig an der Nadelschutzhülse befestigt ist und an dem anderen Ende das Eingriffsglied aufweist. Der Arm kann sich in Längsrichtung der Nadelschutzhülse oder der Vorrichtung und bevorzugt vom proximalen Ende der Nadelschutzhülse in proximale Richtung erstrecken. Insbesondere sind Nadelschutzhülse, Arm und Eingriffsglied einteilig, wie z. B. ein durch Spritzgussformen hergestelltes Kunststoffteil.

Vorzugsweise ist das Eingriffsglied der Nadelschutzhülse in ihrer Ausgangsposition oder einer Position, in der sie aus der Ausgangsposition in distale Richtung bewegt ist, in einem Eingriff mit einem in distale Richtung wirkenden Anschlag. Der in distale Richtung wirkende Anschlag kann auch als Endanschlag angesehen werden, da er eine Bewegung der Nadelschutzhülse in distale Richtung sperrt. Insbesondere befindet sich die Nadelschutzhülse in dieser Position oder der Ausgangsposition in einer ausgefahrenen Position. Insbesondere kann eine Bewegung der Nadelschutzhülse aus der ausgefahrenen Position in proximale Richtung grundsätzlich möglich sein.

In bevorzugten Ausführungsformen ist das federnd angeordnete Eingriffsglied bei einer Bewegung der Nadelschutzhülse aus der ersten ausgefahrenen Position in proximale Richtung von einer Führungsfläche auslenkbar, wobei das Eingriffsglied in oder bei Erreichen der eingefahrenen Position der Nadelschutzhülse aus der ausgelenkten Position zurückfedert. Sobald das Eingriffsglied zurückgefedert ist, kann die Nadelschutzhülse nicht mehr in die erste ausgefahrene Position, sondern nur noch in die zweite ausgefahrene Position bewegt werden. Allgemein bevorzugt ist, dass die Nadelschutzhülse nur noch in die zweite ausgelenkte Position bewegbar ist, wenn die Nadelspitze über das distale Ende der Nadelschutzhülse hervorgetreten ist oder/und die Nadelschutzhülse bis in ihre eingefahrene Position, insbesondere in einen Anschlag mit dem Gehäuse, zurückgeschoben wurde. Insbesondere kann das Eingriffsglied quer zur Längsachse der Nadelschutzhülse, insbesondere windschief zur Längsachse federn.

In bevorzugten Ausführungen kann das Eingriffsglied bei einer Bewegung der Nadelschutzhülse aus ihrer eingefahrenen Position in distale Richtung, d. h. bei der Bewegung in die zweite ausgefahrene Position, von einer, insbesondere weiteren Führungsfläche auslenkbar sein, wobei das Eingriffsglied in der ausgefahrenen Position der Nadelschutzhülse aus der ausgelenkten Position zurückfedert. Insbesondere ist das Eingriffsglied quer zur Längsachse insbesondere windschief auslenkbar und zurückfederbar.

Besonders bevorzugt ist das Eingriffsglied in der zweiten ausgefahrenen Position der Nadelschutzhülse zwischen einem in distale Richtung wirkenden Anschlag, der eine Bewegung der Nadelschutzhülse in distale Richtung sperrt und insbesondere auch als Endanschlag bezeichnet werden kann, und einem in proximale Richtung wirkenden Anschlag, der eine Bewegung der Nadelschutzhülse in proximale Richtung sperrt. Hierdurch wird die Nadelschutzhülse an einer Bewegung in distale und in proximale Richtung gehindert und relativ zum Gehäuse verriegelt.

Allgemein bevorzugt ist, dass wenigstens einer, bevorzugt jeder der Anschläge für das Eingriffsglied und/oder wenigstens eine, bevorzugt jede der Führungsflächen für das Eingriffsglied von einer Führungsbahn gebildet ist. Die Führungsbahn kann eine Führungskurve umfassen oder sein und/oder eine Führungsgerade umfassen oder sein. Die Führungsbahn wird bevorzugt von dem Gehäuse gebildet. Insbesondere ist die Führungsbahn eine zur Innenseite des Gehäuses offene Ausnehmung, die z. B. nutförmig sein kann. Die Nut kann eine Sacknut mit einem Nutgrund oder eine durch die Wand des Gehäuses durchgehende Nut oder Ausnehmung sein. Beispielsweise kann die durchgehende Nut von außen sichtbar sein, wobei die Sacknut von außen nicht sichtbar sein kann. Der wenigstens eine Anschlag und/oder die wenigstens eine Führungsfläche kann bei einer Sacknut von dem Nutgrund und/oder den Nutflanken oder bei einer durchgehenden Nut von den Nutflanken gebildet werden. Das vorzugsweise nockenförmige Eingriffsglied greift in die Führungsbahn, insbesondere von der Innenseite des Gehäuses her. Alternativ kann das Eingriffsglied von der Außenseite des Gehäuses her in die Führungsbahn eingreifen, die dann eine zur Außenseite des Gehäuses offene Ausnehmung ist. Diese alternative Anordnung schützt insbesondere davor, dass das Eingriffsglied manuell entsichert werden kann. Das Eingriffsglied kann lediglich bei einem Teil der Verschiebebewegungen der Nadelschutzhülse in die Führungsbahn oder Nut eingreifen. Bevorzugt wird jedoch, dass das Eingriffsglied während allen Verschiebebewegungen der Nadelschutzhülse in die Führungsbahn oder Nut eingreift.

In optionalen Ausführungen kann das Eingriffsglied in der Ausgangsposition der Nadelschutzhülse in einem Eingriff mit einem in distale Richtung wirkenden Anschlag, zum Beispiel des Gehäuses, sein. Der Eingriff ist so oder das Eingriffsglied und der Anschlag sind so gebildet, dass eine Bewegung der Nadelschutzhülse aus ihrer Ausgangsposition in Richtung ihrer ausgefahrenen Position nur durch Aufbringung einer in distale Richtung wirkenden Kraft auf die Nadelschutzhülse, insbesondere zusätzlich zu der durch die vorgespannte Feder erzeugten Kraft lösbar ist. Bei dem Anschlag handelt es sich nicht um einen Endanschlag, der jegliche Bewegung der Nadelschutzhülse in axiale Richtung verhindert, sondern um einen lösbaren Anschlag oder Zwischenanschlag, der eine Bewegung der Nadelschutzhülse in distale Richtung erschwert. Das Eingriffsglied kann bei einer hinreichend großen Kraft über den Anschlag schnappen oder/und an dem Anschlag vorbeibewegt werden. Mit anderen Worten ist der Eingriff mit einer Kraft, die gegenüber der für Verschiebebewegung der Nadelschutzhülse erforderlichen Kraft erhöht ist, lösbar.

Besonders bevorzugt ist die Nadelschutzhülse so an der Vorrichtung angeordnet, dass eine Bewegung der Nadelschutzhülse aus ihrer Ausgangsposition in Richtung ihrer ausgefahrenen Position durch Aufbringen einer in distale Richtung wirkenden Kraft, insbesondere zusätzlich zu der von der vorgespannten Feder auf die Nadelschutzhülse ausgeübten Kraft, bewegbar ist. Das Federelement ist besonders bevorzugt so konfiguriert, dass die von ihr auf die Nadelschutzhülse ausgeübte Kraft kleiner ist als die Kraft, die für eine Bewegung der Nadelschutzhülse aus ihrer Ausgangsposition in Richtung ausgefahrener Position notwendig ist. Dadurch kann verhindert werden, dass sich die Nadelschutzhülse aus ihrer Ausgangsposition aufgrund der Federkraft in die ausgefahrene Position bewegt. Erst wenn zusätzlich die Muskelkraft auf die Nadelschutzhülse ausgeübt wird, wird die Haltekraft der Nadelschutzhülse in ihrer Ausgangsposition überwunden, so dass die Nadelschutzhülse in distale Richtung bewegt wird.

Die erfindungsgemäße Vorrichtung kann optional auch zum Entfernen einer Nadelschutzkappe von einer Nadel dienen. Die Nadelschutzhülse kann so mit der Nadelschutzkappe gekoppelt oder koppelbar sein, dass mit einer Bewegung der Nadelschutzhülse die Nadelschutzkappe von der Nadel entfernbar ist. Dies ist von Vorteil, da die Nadelschutzkappe in bestimmten Einbaulagen vom Verwender der Vorrichtung nicht oder nur schwer greifbar ist, wodurch das Entfernen der Nadelschutzkappe erschwert ist. Die Erfindung erleichtert das Entfernen der Nadelschutzkappe, da der Verwender auch bei kaum oder nicht zugänglicher Nadelschutzkappe diese von der Nadel entfernen kann.

Im Gegensatz zu technischen Lösungen mit einer am distalen Ende der Vorrichtung befestigten Kappe, die vor dem Gebrauch der Vorrichtung von dem distalen Ende entfernt wird und dabei die Nadelschutzkappe mitnimmt, verbleibt bei der Erfindung die Nadelschutzhülse an der Vorrichtung.

In den optionalen Ausführungen ist die Nadelschutzkappe mittels einer Bewegung der Nadelschutzhülse relativ zum Gehäuse in Richtung einer ausgefahrenen Position der Nadelschutzhülse von der Nadel entfernbar. Insbesondere kann die Nadelschutzhülse aus einer optionalen Ausgangsposition der Nadelschutzhülse, welche die Nadelschutzhülse in einem Auslieferungszustand oder Ausgangszustand der Vorrichtung einnimmt, in die ausgefahrene Position bewegbar sein. Die Ausgangsposition kann die eingefahrene Position oder eine Position sein, in der die Nadelschutzhülse axial zwischen der eingefahrenen Position und einer ausgefahrenen Position der Nadelschutzhülse angeordnet ist.

Bevorzugt ist die Nadelschutzhülse durch den Verwender der Vorrichtung, insbesondere aus der Ausgangsposition der Nadelschutzhülse, aus dem Gehäuse in Richtung ausgefahrener Position herausziehbar, insbesondere mit einer Bewegung in distale Richtung. Die Nadelschutzhülse kann die Nadelschutzkappe in ihrem Ausgangszustand und während der Bewegung in die ausgefahrene Position umgeben. Beim Herausziehen der Nadelschutzhülse kann die Nadelschutzkappe von der Nadel abgestreift oder entfernt werden. Insbesondere kann die Nadelschutzhülse die Nadelschutzkappe mitnehmen. Insbesondere findet beim Herausziehen der Nadelschutzhülse aus dem Gehäuse zum Entfernen oder Abstreifen der Nadelschutzkappe keine oder keine wesentliche Relativbewegung zwischen Nadelschutzkappe und Nadelschutzhülse statt. Das Herausziehen der Nadelschutzkappe kann ausschließlich oder anteilig mit Muskelkraft des Verwenders stattfinden. Der Verwender kann die Nadelschutzhülse greifen und sie aus dem Gehäuse herausziehen. Die Nadelschutzhülse kann hierzu ein Greifelement, wie z. B. eine strukturierte Oberfläche oder eine insbesondere ringförmig um die Nadelschutzhülse umlaufende und nach außen gerichtete Abragung aufweisen. Z. B. kann eine solche Abragung auch für einen Anschlag dienen, in den sie in der eingefahrenen Position der Nadelschutzhülse mit z. B. dem distalen Ende des Gehäuses gerät.

Die Vorrichtung kann ferner ein Eingriffselement aufweisen, das an oder in die Nadelschutzkappe greift. Die Nadelschutzhülse und das Eingriffselement sind bevorzugt so gekoppelt, dass die Nadelschutzkappe die Bewegung der Nadelschutzhülse in ihre ausgefahrene Position mitmacht. Hierdurch kann die Nadelschutzkappe von der Nadel abgestreift werden. Das Eingriffselement kann z. B. ein separates Teil sein, das mit der Nadelschutzhülse gekoppelt ist, wie z. B. ein Metallring, der seitlich in die Nadelschutzkappe eingreift, insbesondere durch Materialverformung bei der Nadelschutzkappe. Besonders bevorzugt ist das Eingriffselement einteilig mit der Nadelschutzhülse. Das Eingriffselement ist bevorzugt an einem Ende eines Arms angeordnet, der mit einem anderen Ende an der Nadelschutzhülse befestigt ist. Der Arm kann sich in Längsrichtung der Nadelschutzhülse oder der Vorrichtung und bevorzugt vom proximalen Ende der Nadelschutzhülse in proximale Richtung erstrecken. Auch hier ist es von Vorteil, die Nadelschutzhülse im Spritzguss aus Kunststoff zu formen. Insbesondere kann das Eingriffselement hakenförmig sein, so dass es in oder an die Nadelschutzkappe greift. Besonders bevorzugt greift das Eingriffselement in die Flanke der Nadelschutzkappe oder an das proximale Ende der Nadelschutzkappe, insbesondere zwischen Nadelschutzkappe und einem Medikamentenabschnitt des Produktbehältnisses. Insbesondere kann als Medikamentenabschnitt derjenige Abschnitt des Produktbehältnisses definiert werden, in dem der Kolben verschiebbar ist oder/und in dem das Medikament aufgenommen ist. Bei der Bewegung der Nadelschutzhülse aus ihrer Ausgangsposition in die ausgefahrene Position wird über den Eingriff des mindestens einen Eingriffselements die form- und/oder kraftschlüssig an dem Nadelträger oder dem Befestigungsabschnitt befestigte Nadelschutzkappe von der Nadel bzw. von dem Nadelträger oder dem Befestigungsabschnitt abgestreift. Bevorzugt bleibt der Eingriff des mindestens einen Eingriffselements mit der Nadelschutzkappe bestehen, bis die Nadelschutzhülse von dem Nadelträger oder dem Befestigungsabschnitt gelöst ist.

Bevorzugt können neben dem Eingriffsmittel wenigstens ein oder alle aus Eingriffsglied und Eingriffselement einteilig an der bevorzugt als Spritzgussteil gefertigten Nadelschutzhülse gebildet sein.

Optional ist das Eingriffsglied der Nadelschutzhülse in der ersten ausgefahrenen Position in einem Eingriff mit einem in proximale Richtung wirkenden Anschlag oder in einen solchen Eingriff bringbar, der eine Bewegung der Nadelschutzhülse in proximale Richtung erschwert, wobei der Eingriff durch Aufbringung einer auf die Nadelschutzhülse und in proximale Richtung wirkenden Kraft lösbar ist. Insbesondere kann das Eingriffsglied über den Anschlag schnappen oder/und an dem Anschlag vorbeibewegt werden, wenn eine hinreichend große in proximale Richtung wirkende Kraft auf die Nadelschutzhülse ausgeübt wird. Beispielsweise ist die hinreichend große Kraft deutlich größer als die Kraft, welche das Federelement auf die Nadelschutzhülse ausübt.

Die Erfindung eröffnet auch Wege für eine sichere Verabreichung von flüssigen Medikamenten, deren Wirkstoff(e) an Luft der Gefahr der Kontamination unterliegt, wie insbesondere Formulierungen, die als wesentlichen Wirkstoff FSH oder eine FSH Variante enthalten, oder eines anderen hierin genannten Wirkstoffs oder Medikaments. Dabei ist es ein besonderes Anliegen, den Einsatz von Konservierungsmitteln zu vermeiden, nicht zuletzt im Hinblick auf negative Auswirkungen auf den oder die jeweiligen Wirkstoff(e), aber auch auf die zusätzlichen Kosten, die durch die Beimischung von Konservierungsmitteln entstehen. Nach der Erfindung wird das Produktbehältnis, das auch als Reservoir bezeichnet werden kann, mit dem nicht konservierten flüssigen Medikament an den Patienten abgegeben, vorzugsweise in Form eines kompletten Verabreichungsgeräts oder eines mit einfachen Handgriffen montierbaren Geräteteils. Das nicht konservierte, bei Anwesenheit von Luft kontaminationsgefährdete Medikament ist in dem bis unmittelbar vor Verabreichung steril geschlossenen Reservoir sicher aufbewahrt, so dass es in diesem Zustand über einen Zeitraum von mehreren Tagen oder durchaus auch Monaten oder Jahren aufbewahrt werden kann. Die Sterilität wird erst unmittelbar vor Gebrauch vom Patienten oder einem verabreichenden Arzt aufgehoben.

Bevorzugt ist ein Verabreichungsgerät mit Ablaufsteuerung, dessen Reservoir steril geschlossen ist und ein flüssiges Medikament in Form beispielsweise einer flüssigen Formulierung von FSH oder einer FSH Variante oder ein anderes hierin genanntes Medikament enthält. Die Verwendung von Karpulen oder Spritzen, die ein nicht konserviertes Medikament, beispielsweise FSH oder eine nicht konservierte FSH Variante, in sterilem und daher auch ohne Konservierungsmittel haltbaren Zustand enthalten, ist aber auch als solche ein Gegenstand der Erfindung. Bevorzugt ist auch ein Verabreichungsgerät ohne Dosierfähigkeit, also ohne Dosierglied, obgleich auch für die Verabreichung solcher Medikamente die Möglichkeit der individuellen Dosierung durch den Patienten selbst von großem Vorteil ist. Weitere Medikamente, die nach der Erfindung in nicht konserviertem Zustand in einem steril verschlossenen Reservoir des Verabreichungsgeräts und somit praktisch zeitlich unbegrenzt im Handel, beim Arzt oder auch bereits beim jeweiligen Patienten aufbewahrt werden können, sind beispielsweise Neuroleptica (Fluphenazini decanoas), gefäßerweiternde Mittel (Adrenalinum), Blutprodukte (Etamsylat, Epoetin alfa, Filgrastim (G-CSF), Nadroparinum calcium, Desmopressini acetas), Medikamente gegen rheumatische Erkrankungen (Methotrexat, Etanerceptum), Onkologica (Cladribinum, Interferonum humanum gamma-1b ADN) und Medikamente gegen Infektionskrankheiten (Herpes simplex Typ1/Typ2, humanes Immunglobulin). In Klammern sind übliche oder bevorzugte Wirkstoffe für die jeweilige Medikamentengruppe angegeben.

Ein FSH basiertes Medikament enthält vorzugsweise eine alpha- und eine beta-Untereinheit. Die Proteine für die verwendbaren Formulierungen können durch verschiedene Verfahren gewonnen werden. Vorzugsweise ist das einzusetzende FSH ein Heterodimer, das eine α-Untereinheit und eine β-Untereinheit umfasst, wie sie in der EP 1 188 444 A1 näher beschrieben werden. Vorzugsweise wird mit der erfindungsgemäßen Vorrichtung eine Formulierung von FSH und/oder einer FSH-Variante in einem wässrigen Lösungsmittel verabreicht. Der Ausdruck "wässriges Lösungsmittel" bezieht sich auf ein flüssiges Lösungsmittel, das Wasser enthält. Wässrige Lösungsmittelsysteme können nur aus Wasser bestehen oder können aus Wasser und einem oder mehreren mischbaren Lösungsmitteln bestehen und können weitere gelöste Bestandteile (Solute) enthalten, wie beispielsweise Zucker oder andere Hilfsstoffe. Die herkömmlich verwendeten mischbaren Lösungsmittel sind die kurzkettigen organischen Alkohole, wie Methanol, Ethanol, Propanol, kurzkettige Ketone, wie Aceton, und Polyalkohole, wie Glycerin. Bevorzugt besteht das Lösungsmittelsystem nur aus Wasser für Injektionszwecke. Hilfsstoffe können ausgewählt sein aus Isotonizitätsmitteln, Konservierungsstoffen, Puffersystemen, insbesondere Phosphatpuffern, Thioetherverbindungen wie Methionin als Antioxidationsmittel, Dispergiermitteln bzw. Emulgatoren wie Poloxameren und deren Gemischen. Die Formulierung enthält keine Konservierungsstoffe, insbesondere keinen Konservierungsstoff der Gruppe, bestehend aus Phenol, m-Cresol, Chlorcresol, einem Paraben, das ausgewählt ist aus Methyl-, Ethyl-, Propyl- oder Butylparaben, Benzalkoniumchlorid, Benzethoniumchlorid, Natriumdehydroacetat, Benzylakohol und Thimerosal. Bevorzugt ist weiterhin, wenn die Formulierung frei ist von Poly-, Oligo- und Dicarbonsäuren sowie Glycin und/oder Glycerin.

Insbesondere für die Verabreichung von nicht konservierten flüssigen Medikamenten, wie die Verabreichung flüssiger Formulierungen von FSH oder einer FSH Variante, ist es von Vorteil, wenn im steril geschlossenen Reservoir nur eine Medikamentenmenge aufbewahrt wird, die höchstens so groß wie eine Tagesdosis, noch bevorzugter wie eine Maximaldosis (Monodosis) ist, in der das betreffende Medikament in einer einzigen Verabreichung von höchstens einigen Minuten Dauer, vorzugsweise einer einzigen Injektion, verabreicht wird.

Das Reservoir und somit auch das Verabreichungsgerät sind daher vorzugsweise ein Reservoir und ein Verabreichungsgerät für eine Monodosis des Medikaments. Die Maximaldosis für Medikamente auf der Basis von beispielsweise FSH oder einer FSH Variante ist typischerweise 300 IU, in Ausnahmefällen 500 IU, gemessen über eine Vielzahl von Patienten. Die im Reservoir befindliche Medikamentenmenge entspricht im Falle solch eines Medikaments daher in bevorzugten Ausführungen höchstens einer Dosis von 500 IU, bevorzugter höchstens 300 IU. Da eine Mehrzahl der Patienten mit deutlich geringeren Dosen pro Einzelverabreichung auskommt, beispielsweise mit höchstens 200 IU oder auch nur höchstens 100 IU, kann die Füllmenge in solch einer Therapie vorteilhafterweise auch höchstens dieser Dosis entsprechen.

In einer Weiterentwicklung werden die Verabreichungsgeräte jeweils mit einem Reservoir angeboten, das eine dem tatsächlichen Bedürfnis des jeweiligen Patienten genauer angepasste Füllmenge aufweist. So können baugleiche Verabreichungsgeräte beispielsweise in zwei, drei oder mehr unterschiedlichen Füllmengenvarianten zum Einsatz gelangen. Im Falle beispielsweise einer FSH-basierten Therapie können Verabreichungsgeräte der gleichen Bauart angeboten werden, die sich im Wesentlichen nur hinsichtlich der Füllmenge ihres Reservoirs voneinander unterscheiden, beispielsweise ein Gerät mit einem Reservoir, das 300 IU des Medikaments, ein weiteres Gerät, das 200 IU des gleichen Medikaments und noch ein Gerät, das 100 IU des Medikaments enthält, jeweils im sterilen Zustand. Es kann auch eine nochmalige Unterteilung in Zwischenstufen vorgenommen werden, beispielsweise in Form einer vierten Füllmengenvariante mit einem Reservoir, dass 150 IU des Medikaments im sterilen Zustand enthält. Im Allgemeinen können sich Dosen für FSH zwischen 75 IU bis 300 IU, in Ausnahmefällen bis 500 IU erstrecken. Das Gerät ist vorzugsweise jeweils für den einmaligen Gebrauch konzipiert, als Einwegartikel, und kann vorteilhafterweise über den Hausmüll entsorgt werden. Erst unmittelbar vor der Verabreichung wird das Medikament im bis zu diesem Zeitpunkt steril geschlossenen Reservoir mit der äußeren Umgebung in Kontakt gebraucht.

Für den sterilen Verschluss des Reservoirs kommen insbesondere zwei Varianten in Betracht. In der ersten Variante bildet eine Karpule das Reservoir, die für die Verabreichung einen Auslass aufweist, der von einem durchstechbaren Septum steril verschlossen wird. Das Medikament ist in derartigen Ausführungen zwischen dem Septum und einem axial beweglichen Kolben steril eingeschlossen. Mit einem derartigen Reservoir kommt eine Nadeleinheit zum Einsatz mit einem Nadelhalter und einer den Nadelhalter durchragenden Injektionsnadel. Der Nadelhalter dient der Halterung der Injektionsnadel und der Herstellung des Fluidanschlusses der Nadel an das Reservoir. Für den Anschluss kann unmittelbar das Reservoir oder vorzugsweise das Gehäuse einen Verbindungsabschnitt im Bereich des Auslasses des Reservoirs aufweisen. Der Verbindungsabschnitt und ein Verbindungsabschnitt des Nadelhalters sind kooperierend ausgebildet, so dass bei Herstellung der mechanischen Verbindung zwischen dem Nadelhalter und dem Verbindungsabschnitt des Reservoirs oder des Gehäuses die Injektionsnadel das Septum durchsticht. Nach dem Durchstechen ist der Innenraum des Reservoirs über die Nadel mit der Umgebung verbunden. Im Anschluss an die Herstellung dieser Fluidverbindung wird möglichst ohne Verzug das Medikament verabreicht. Anschließend wird das Verabreichungsgerät oder zumindest ein das vollständig oder teilweise entleerte Reservoir enthaltender Teil des Geräts, gegebenenfalls auch nur das Reservoir, entsorgt. In der zweiten Variante bildet eine Spritze oder spritzenähnliches Gebilde das Reservoir. Im Unterschied zur ersten Variante weist das Reservoir der zweiten Variante an seinem distalen Ende bereits eine Injektionsnadel auf, so dass von Hause aus bereits die Fluidverbindung zwischen dem Reservoir und einem stromabwärtigen Auslass der Injektionsnadel besteht. Für den sterilen Verschluss sorgt ein externes Dichtelement, wie die hierin beschriebene Nadelschutzkappe, das den Nadelauslass steril verschließt. Das Dichtelement wird unmittelbar vor Gebrauch abgezogen.

In einer einfachsten Ausführung, in der das Medikament beispielsweise bereits in exakt der Dosis im Reservoir enthalten ist, die mittels einer einzigen Verabreichung ausgeschüttet werden soll, kann auf das Merkmal der Dosierbarkeit verzichtet werden, das Dosierglied kann dementsprechend entfallen. Auch in derartigen Ausführungen ist es von Vorteil, wenn das Verabreichungsgerät über eine Primingfunktion verfügt. Die Primingfunktion kann vom Förderglied erfüllt werden, indem dieses im Ausgangszustand des Verabreichungsgeräts in einem lösbaren Blockiereingriff blockiert ist, aus dem es durch Aufbringung einer Kraft oder eines Drehmoments bewegt werden muss. Durch den kurzen Priminghub wird ein durch die Länge des Priminghubs vorgegebenes Volumen aus dem Reservoir ausgestoßen. Das Ende des Priminghubs wird vorzugsweise durch einen Priminganschlag bestimmt. Im Kontakt mit dem Priminganschlag kann das Förderglied danach die Ausschüttbewegung ausführen. Die Ausschüttbewegung ist vorzugsweise eine Axialbewegung in eine Vortriebsrichtung. Der Priminghub ist vorzugsweise ebenfalls eine Axialbewegung in die Vortriebsrichtung. Zwischen dem Priminghub und dem Ausschütthub ist vorteilhafterweise eine Querbewegung des Förderglieds erforderlich und auch erst nach Ausführung des Priminghubs möglich, wodurch das Primen und das Ausschütten sicher voneinander getrennt werden.

Auch für Therapien mit nicht konserviertem Medikament oder einem für nur eine einzige Verabreichung befüllten Reservoir, einer Monodosis, ist es von Vorteil, wenn das Verabreichungsgerät über die Fähigkeit der Dosierbarkeit verfügt, also das zuvor genannte Dosierglied aufweist. Auf diese Weise kann trotz einer optionalen Vielfalt von Varianten hinsichtlich der Füllmenge für jeweils nur eine einzige Verabreichung in jeder Variante nochmals die Dosis genauer eingestellt werden. Es kann mit anderen Worten durch die Bereitstellung mehrerer Füllmengenvarianten die Medikamentenmenge über viele Patienten gesehen insgesamt verringert werden, nämlich im Vergleich zu Ausführungen, in denen das Reservoir jeweils die Maximaldosis enthält, die innerhalb der Therapie überhaupt in einer einzigen Verabreichung ausgeschüttet wird. Andererseits kann der einzelne Patient innerhalb der passend gewählten Füllmengenvariante nochmals individuell die auf ihn zugeschnittene Dosis einstellen und auch nur diese sich verabreichen.

Die Erfindung wurde anhand mehrerer Ausführungen erläutert. Nachfolgend werden besonders bevorzugte Ausführungen der Erfindung anhand von Figuren erläutert. Die dabei offenbar werdenden Merkmale bilden jeweils einzeln und in jeder Merkmalskombination die Gegenstände der Ansprüche und auch die vorstehend beschriebenen Ausgestaltungen vorteilhaft weiter. Es zeigen:
- Figur 1: ein Verabreichungsgerät in einem Ausgangs- oder Auslieferungszustand mit einer aufgesetzten Nadelschutzkappe,
- Figuren 2a, 2b: das Verabreichungsgerät aus Figur 1 mit abgenommener Nadelschutzkappe vor einem Primen und mit einer Nadelschutzhülse in Ausgangsposition,
- Figur 3a, 3b: das Verabreichungsgerät aus Figur 1 nach dem Primen und Einstellen einer Dosis und mit der Nadelschutzhülse in eingefahrener Position,
- Figur 4a, 4b: das Verabreichungsgerät nach dem Ausschütten einer Dosis und mit der Nadelschutzhülse in einer Endposition,
- Figur 5: einen Dosierabschnitt des Verabreichungsgeräts aus Figur 1 in einem Querschnitt,
- Figur 6: den Bereich eines Dosieranzeigefensters der Verabreichungsvorrichtung aus Figur 1 in einem Querschnitt,
- Figur 7: den Bereich eines Dosierrastelements der Verabreichungsvorrichtung aus Figur 1,
- Figur 8a-8d: eine alternative Führungsbahn für das Verabreichungsgerät aus Figur 1, und
- Figur 9: ein mit einer zusätzlichen Hülse am proximalen Ende des Gehäuses modifiziertes Verabreichungsgerät.

Bei dem in den Figuren dargestellten Verabreichungsgerät handelt es sich um einen langgestreckten Injektionspen. Das Verabreichungsgerät ist für den einmaligen Gebrauch bestimmt, für eine einzige Injektion eines flüssigen Medikaments, das insbesondere eine flüssige Formulierung auf der Basis von FSH oder einer FSH Variante für eine Hormonbehandlung zur Stimulierung der Eierstöcke und daraus folgend durch Befruchtung der stimulierten Eizellen der Schwangerschaft sein kann. Das flüssige Medikament, d.h. die flüssige Formulierung, ist nicht konserviert, enthält also keine Konservierungsmittel. Handelt es sich wie bevorzugt um eine Formulierung, die FSH oder eine FSH Variante enthält, muss das Medikament nach Kontakt mit der Umgebungsluft daher innerhalb weniger Minuten, allenfalls innerhalb weniger Stunden, verabreicht werden.

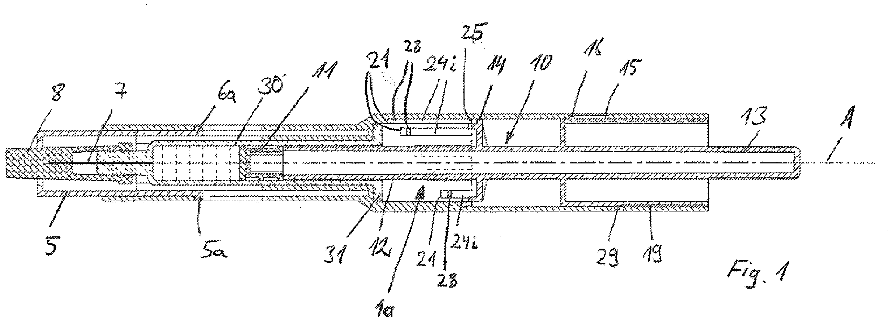

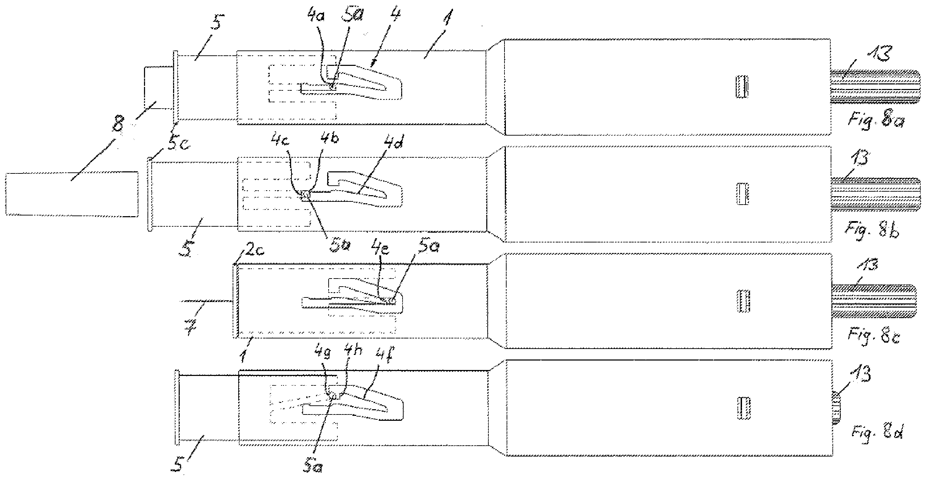

Die Figuren 1 und 8a zeigen Verabreichungsgeräte in einem Ausgangs- oder Auslieferungszustand vor der Benutzung, wobei das Verabreichungsgerät aus Figur 8a mit einer alternativen Führungsbahn 4 ausgestattet ist. In diesem Zustand erhält der Patient das Gerät. Das Gerät umfasst ein hülsenförmiges Gehäuse 1 mit einem vorderen, distalen Gehäuseabschnitt, in dem ein das Medikament in sterilem Zustand enthaltendes Reservoir 30 aufgenommen ist, und einen hinteren, proximalen Gehäuseabschnitt, in dem Strukturen geformt sind, die mit einer Dosier- und Fördereinrichtung des Geräts zusammenwirken, um dem Patienten eine individuelle Auswahl einer zu verabreichenden Dosis des Medikaments zu ermöglichen. Der proximale Gehäuseabschnitt kann bei der Verabreichung auch als Griffteil dienen. Von der Dosier- und Fördereinrichtung ist ein Bedienelement 13 erkennbar, das in proximaler Richtung am proximalen Ende aus dem Gehäuse 1 ragt.

Im Gehäuse 1 sind ein Dosisanzeigefenster 2 und ein Reservoirfenster 3 geformt. Durch das Fenster 2 ist die eingestellte Dosis ablesbar, und durch das Fenster 3 ist das Reservoir 30 erkennbar. Das Reservoir 30 weist am distalen Ende einen Verbindungsabschnitt auf, an dem eine Nadel 7 unlösbar befestigt ist. Eine an dem Reservoir 30 befestigte Nadelschutzkappe 8 wird erst unmittelbar vor der Verabreichung von der Nadel 7, um bis zu diesem Zeitpunkt die Sterilität des Medikaments zu gewährleisten.

Die Figuren 1 und 8a zeigen das Verabreichungsgerät wie gesagt im Ausgangszustand mit steril verschlossenem Reservoir 30, genauer gesagt im Lagerungszustand, in dem das Gerät über einen längeren Zeitraum von wenigstens mehreren Monaten gelagert werden kann, da der luftdichte, sterile Verschluss des Reservoirs 30 die Haltbarkeit auch von nicht konservierten Medikamenten sicherstellt. Um sich das Medikament zu verabreichen, entfernt der Patient unmittelbar vor der Verabreichung die Nadelschutzkappe 8 von der Nadel 7. Beim Beispiel aus Figur 1 greift der Patient die Nadelschutzkappe 8 und zieht sie von der Nadel 7 ab. Beim Beispiel aus den Figuren 8a bis 8d entfernt der Patient die Nadelschutzkappe 8 mit Hilfe einer Nadelschutzhülse 5.

Mit Bezug auf die Figuren 8a und 8b wird eine Einrichtung beschrieben, mit deren Hilfe die Nadelschutzkappe 8 von der Nadel 7 entfernbar ist. Diese Einrichtung kann als Zusatzausstattung zu der Vorrichtung aus den Figuren 1 bis 7 Anwendung finden. In der in Figur 8a gezeigten Ausgangsposition der Nadelschutzhülse 5, welche die Nadelschutzhülse 5 im Auslieferungszustand der optionalen Injektionsvorrichtung in Bezug auf das Gehäuse 1 einnimmt, greift die Nadelschutzhülse 5 z. B. mit mindestens einem Eingriffselement an oder in die Nadelschutzkappe 8, z. B. zwischen einem Medikamentenabschnitt des Produktbehältnisses und dem proximalen Ende der Nadelschutzkappe 8 oder seitlich in die Nadelschutzkappe 8.

Die Nadelschutzhülse 5 weist einen sich vom proximalen Ende der Nadelschutzhülse 5 in proximale Richtung erstreckenden Arm auf, an dessen proximalen Ende ein Eingriffsglied 5a gebildet ist, das in eine Führungsbahn 4, die durch das Gehäuse 1 gebildet wird, eingreift. Durch die Anordnung an dem Arm ist das Eingriffsglied 5a quer zur Längsachse der Vorrichtung federnd, während es entlang der Längsachse relativ zur Nadelschutzhülse 5 im Wesentlichen feststeht. Das Eingriffsglied 5a ist nockenförmig und weist radial nach außen in die zur Innenseite des Gehäuses 1 hin offene Führungsbahn 4.

An der Flanke der nutförmigen Führungsbahn 4 ist ein in distale Richtung wirkender Anschlag 4a für einen Eingriff mit dem Eingriffsglied 5a gebildet. Der Eingriff ist so gestaltet, dass er nur mit der Kraft des vorgespannten Federelements 6 nicht lösbar ist.

Durch Aufbringen einer zusätzlichen Kraft, insbesondere einer Muskelkraft des Verwenders, auf die Nadelschutzhülse 5 in distale Richtung wird der Eingriff mit dem Anschlag 4a gelöst, indem das Eingriffsglied 5a über den Anschlag 4a schnappt. Hierdurch wird die Nadelschutzhülse 5 aus ihrer Ausgangsposition in distale Richtung in ihre erste ausgefahrene Position und an dem Anschlag 4a vorbei bewegt. Um unter anderem die Greifbarkeit der Nadelschutzhülse 5 für den Verwender zu verbessern, weist die Nadelschutzhülse 5 eine ringförmig radial nach außen weisende Abragung 5c auf.

Das Eingriffsglied 5a schlägt am Ende der Bewegung der Nadelschutzhülse 5 in ihre erste ausgefahrene Position an einem Endanschlag 4c an, der in distale Richtung wirkt, jedoch eine Nadelschutzhülse in distale Richtung auch bei erhöhtem Kraftaufwand sperrt.

Bei der Bewegung in distale Richtung nimmt die Nadelschutzhülse 5 über das mindestens eine Eingriffselement die Nadelschutzkappe 8 mit, so dass diese von dem Produktbehältnis bzw. dem Befestigungsabschnitt gelöst oder abgestriffen wird. Die Nadelschutzkappe 8 kann somit von der Vorrichtung entfernt werden (Figur 8b).

In der ersten ausgefahrenen Position der Nadelschutzhülse 5, die in Figur 8b gezeigt wird, wird eine Bewegung der Nadelschutzhülse 5 in proximale Richtung, das heißt in eine eingefahrene Position, durch einen Eingriff des Eingriffsglieds 5a an den Anschlag 4b erschwert. Durch Aufbringung einer in proximale Richtung wirkenden Kraft, die z. B. deutlich größer ist als die Kraft, die benötigt werden würde, wenn die Nadelschutzhülse 5 nur gegen die Federkraft des Federelements 6 geschoben werden müsste, schnappt das Eingriffsglied 5a über den Anschlag 4b, wodurch die an ihrem distalen Ende an die Injektionsstelle angepresste Nadelschutzhülse 5 ruckartig in das Gehäuse 1 verschoben wird, bis die Abragung 5c an dem distalen Ende des Gehäuses 1 anschlägt. Durch das ruckartige Zurückschieben der Nadelschutzhülse 5, das durch den Eingriff des Eingriffsglieds 5a an den Anschlag 4b erzielt wird, wird die Nadel 7 in den Patienten gestochen. Der übrige Bewegungsablauf des Eingriffsglieds 5a in der Führungsbahn 4 aus den Figuren 8a bis 8d entspricht dem Bewegungsablauf des Eingriffsglieds 5a bei der Vorrichtung aus den Figuren 1 bis 7.

Das Medikament steht nach dem Entfernen der Nadelschutzkappe 8 mit der Umgebung in Verbindung und muss im Falle eines bevorzugt nicht konservierten Medikaments auf der Basis von FSH oder einer FSH Variante innerhalb kurzer Zeit, am besten unverzüglich verabreicht werden.

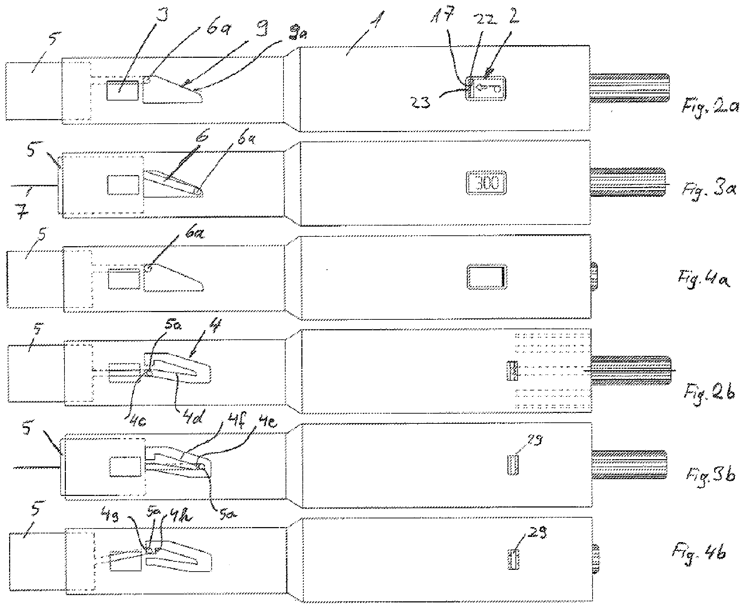

Die Vorrichtung aus den Figuren 1 bis 7 umfasst ebenfalls eine Nadelschutzhülse 5, die von dem Gehäuse 1 gelagert wird. In den Figuren 1, 2a und 2b befindet sich die Nadelschutzhülse in ihrer Ausgangsposition, nämlich in Ihrer ersten ausgefahrenen Position. Die Nadelschutzhülse 5 steht in der ausgefahrenen Position distal über das distale Ende der Nadelspitze.

Wie am besten aus den Figuren 2a bis 4a erkennbar ist, weist die Nadelschutzhülse 5 einen sich vom proximalen Ende der Nadelschutzhülse 5 in proximale Richtung erstreckenden Arm auf, an dessen proximalem Ende ein Eingriffsglied 5a gebildet ist, das in eine Führungsbahn 4, die durch das Gehäuse 1 gebildet wird, eingreift. Durch die Anordnung an dem Arm ist das Eingriffsglied 5a quer zur Längsachse der Vorrichtung federnd, während es entlang der Längsachse relativ zur Nadelschutzhülse 5 im Wesentlichen feststeht. Das Eingriffsglied 5a ist nockenförmig und weist radial nach Außen in die zur Innenseite des Gehäuses 1 hin offene Führungsbahn. Das Eingriffsglied 5a einschließlich Arm 6 ist einteilig an der Nadelschutzhülse 5 gebildet.

Das Eingriffsglied 5a ist in der Ausgangsposition der Nadelschutzhülse 5 im Eingriff mit einem Endanschlag 4c, der in distale Richtung wirkt und eine Bewegung der Nadelschutzhülse 5 in distale Richtung auch bei erhöhtem Kraftaufwand sperrt. Es sei angemerkt, dass dieser Anschlag 4c mit einer sehr hohen Kraft wahrscheinlich zerstört werden könnte, wobei im normalen Gebrauch eine solche Kraft nicht auf die Nadelschutzhülse 5 ausgeübt wird.

In der ersten ausgefahrenen Position der Nadelschutzhülse 5, nämlich ihrer Ausgangsposition, die in den Figuren 2a und 2b gezeigt wird, ist eine Bewegung der Nadelschutzhülse 5 in proximale Richtung möglich. Durch Aufbringen einer in proximale Richtung wirkenden Kraft z. B. durch Anpressen des distalen Endes der Nadelschutzhülse 5 an eine Injektionsstelle wird die Nadelschutzhülse 5 in das Gehäuse 1 verschoben, insbesondere gegen die Kraft eines Federelements 6, das weiter unten genauer erklärt wird. Nach dem Zurückschieben der Nadelschutzhülse 5 in das Gehäuse 1 bzw. in die eingefahrene Position (Figuren 3a und 3b) steht die Nadel 7 entsprechend der gewünschten Injektionstiefe, die bevorzugt für eine subkutane Injektion angepasst ist, über das distale Ende der Nadelschutzhülse 5.

Vom distalen Ende der Nadelschutzhülse 5 ragt ein Arm, der als Federelement 6 dient, in proximale Richtung. Der Arm 6 weist ein nockenförmig und nach außen in eine Kulisse, insbesondere Federkulisse 9 ragendes Eingriffsmittel 6a auf. Das Eingriffsmittel 6a ist im Wesentlichen axial fest zur Nadelschutzhülse 5 und quer zur Längsachse der Nadelschutzhülse 5 federn bewegbar. Das Federelement 6 einschließlich Eingriffsmittel 6a ist einteilig mit der Nadelschutzhülse 5. Beim Zurückschieben der Nadelschutzhülse 5 in das Gehäuse 1 gleitet das Eingriffsmittel 6a an einer quer zur Längsachse der Vorrichtung geneigten Führungsfläche 9a ab, wodurch das Federelement 6 und das Eingriffsmittel 6a quer zur Längsachse der Nadelschutzhülse 5 ausgelenkt werden. Hierdurch wird der Arm 6 mit einem Biegemoment gespannt oder elastisch verformt. Durch die von dem Eingriffsmittel 6a auf die Führungsfläche 9a ausgeübte Kraft bzw. die von der Führungsfläche 9a auf das Eingriffsmittel 6a ausgeübte Reaktionskraft wird ein in distale Richtung wirkender Kraftanteil, der über den Arm 6 an die Nadelschutzhülse 5 geleitet wird erzeugt. Beim Zurückschieben in das Gehäuse 1 wird die Nadelschutzhülse 5 gegen diese Kraft bzw.

Kraftanteil der Feder 6 bewegt. Der Kraftanteil der Feder 6 ist insbesondere abhängig von der Neigung der Führungsfläche 6a in Bezug auf die Längsachse.

Die Führungsbahn 4 weist eine Flanke 4d auf, welche beim Zurückschieben der Nadelschutzhülse 5 aus der ersten ausgefahrenen Position (Figuren 2a und 2b sowie 8b) in die eingefahrene Position (Figuren 3a und 3b sowie 8c), das Eingriffsglied 5a quer zur Längsachse der Vorrichtung auslenkt und den Arm, an dem das Eingriffsglied 5a angeordnet ist, federnd vorspannt. Bei oder vor dem Erreichen der eingefahrenen Position der Nadelschutzhülse 5 schnappt das Eingriffsglied 5a aus seiner ausgelenkten Position zurück. Insbesondere ist der Arm, an dem das Eingriffsglied 5a nach dem Einfahren der Nadelschutzhülse 5 zurückfedert, in einer entspannten Position. Insbesondere befindet sich das zurückgefederte Eingriffsglied 5a in einer axialen Flucht mit einem Umschaltabschnitt 4e der Führungsbahn 4, der eine Auslenkung des Eingriffsglieds 5a zurück in die Richtung, aus der es bei der Einfahrbewegung der Nadelschutzhülse 5 geschnappt ist, verhindert. Ferner ist der Umschaltabschnitt 4e so gebildet, insbesondere mit einer schrägen Fläche, dass, wenn die Nadelschutzhülse 5 aus der eingefahrenen Position in distale Richtung, insbesondere in eine zweite ausgefahrene Position bewegt wird, das Eingriffsglied 5a in die gleiche Richtung ausgelenkt wird, in die das Eingriffsglied 5a beim Einfahren, insbesondere nach dem Passieren der Führungsfläche 4d der Nadelschutzhülse 5 zurückfedert. Bei einer Bewegung der Nadelschutzhülse 5 aus der eingefahrenen Position in die zweite ausgefahrenen Position, die stattfindet, wenn die Nadel 7 aus dem Patienten herausgezogen wird, wobei die vorgespannte Feder 6 die Nadelschutzhülse 5 an der Injektionsstelle gedrückt hält, wird das Eingriffsglied 5a von dem Umschaltelement 4e und einer weiteren Führungsfläche 4f quer zur Längsrichtung der Injektionsvorrichtung ausgelenkt, wodurch der als Feder wirkende Arm, der das Eingriffsglied 5a und die Nadelschutzhülse 5 verbindet, vorgespannt wird.

Am Ende der Bewegung der Nadelschutzhülse 5 in die zweite ausgefahrene Position federt das Eingriffsglied 5a quer zur Längsachse der Vorrichtung zurück, nämlich in die Richtung, aus der das Eingriffsglied 5a bei der Bewegung der Nadelschutzhülse 5 aus ihrer eingefahrenen Position in ihre zweite ausgefahrene Position ausgelenkt wurde. Eine Bewegung der Nadelschutzhülse 5 in distale Richtung wird durch einen in distale Richtung wirkenden Endanschlag 4g verhindert. Eine Bewegung der Nadelschutzhülse 5 in proximale Richtung wird durch einen in proximale Richtung wirkenden Anschlag 4h verhindert. In der zweiten ausgefahrenen Position befindet sich das Eingriffsglied 5a zwischen den Anschlägen 4g und 4h, so dass die Nadelschutzhülse 5 - abgesehen von einem kleinen Spiel - axial fest mit dem Gehäuse 1 verriegelbar ist (Figuren 4a und 4b sowie 8d).

Der besondere Vorteil an der Vorrichtung ist, dass die Nadelschutzhülse 5 einschließlich dem Arm, an dem das Eingriffsglied 2a gebildet ist, und dem Arm 6, der als Feder wirkt und an dem das Eingriffsmittel 6a gebildet ist, einteilig ist, insbesondere aus einem Kunststoff im Spritzgussverfahren hergestellt ist. Dadurch kann der Preis für die Herstellung der Nadelschutzhülse 5 und für die Endmontage gesenkt werden.

Nach Verwendung der Vorrichtung wird diese entsorgt, wobei durch die verriegelte Nadelschutzhülse 5 die Gefahr einer Verletzung an der Nadel 7 verringert ist.

Obgleich das Verabreichungsgerät nur für den einmaligen Gebrauch, also nur für eine einzige Injektion vorgesehen ist und als Einwegartikel nach Gebrauch über den Hausmüll entsorgt wird, weist es dennoch eine ganze Reihe von Merkmalen auf, die üblicherweise nur bei Verabreichungsgeräten zu finden sind, die wiederholt verwendet werden. Zum einen verfügt das Verabreichungsgerät über die Fähigkeit der Dosisauswahl, so dass der Patient die Dosis, die er sich verabreichen möchte, selbst einstellen kann. Das vom Hersteller gefüllte Reservoir 30 enthält eine Maximaldosis des Medikaments, die so bemessen ist, dass sie über eine größere Gruppe potentieller Patienten gesehen für jeden dieser Patienten ausreicht. Personen die pro Verabreichung mit einer geringeren Dosis auskommen, können sich diese geringere Dosis selbst einstellen. Das Gerät wird nach Gebrauch mit der im Reservoir 30 entsprechend noch verbliebenen Restmenge des Medikaments entsorgt. Ferner verfügt das Gerät über die Fähigkeit des Primens, also der Entlüftung des Reservoirs 30. Insbesondere wird bei dem Gerät das Primen durch eine konstruktive Maßnahme erzwungen, d.h. der Patient muss zuerst primen, bevor er eine Dosis einstellen und verabreichen kann. Durch das Primen wird im Reservoir 30 möglicherweise enthaltenes freies Gas, insbesondere Stickstoff oder Luft, aus dem Reservoir 30 verdrängt. Luft kann in der Herstellung beim Befüllen des Reservoirs 30 in das Reservoir 30 gelangen. Diese Luft oder gegebenenfalls ein anderes Gas soll zum einen nicht verabreicht werden, verfälscht zum anderen aber auch die Dosis, da die verabreichte Dosis um die Menge des ohne das Primen im Reservoir 30 befindlichen Gases von der eingestellten Dosis abweichen würde.

Das Verabreichungsgerät umfasst eine Fördereinrichtung 10 mit einem ersten Förderglied 11 und einem zweiten Förderglied 12. Das erste Förderglied 11 ist ein im Reservoir 5 beweglich aufgenommener Kolben 11, der das Reservoir 5 an einem Ende steril abdichtet und im Reservoir 30 in eine Vortriebsrichtung längs einer zentralen Längsachse A des Geräts in Richtung auf den Auslass beweglich ist. Die Injektionsnadel 7 erstreckt sich ebenfalls axial auf der Achse A. Das zweite Förderglied 12 bildet eine Kolbenstange. Das Förderglied 12 ist mit dem Kolben 11 lediglich in axialem Druckkontakt, drückt also den Kolben 11 bei Betätigung des Geräts durch losen Druckkontakt axial in Richtung auf den Auslass des Reservoirs 5. Alternativ kann das Förderglied 12 mit dem Kolben 11 axial fest, bevorzugt in und gegen die Richtung auf den Auslass des Reservoirs 5, verbunden sein.

Das Förderglied 12 bildet gemeinsam mit dem Gehäuse 1 auch eine Dosiereinrichtung. Das Förderglied 12 wird im folgenden daher als Dosier- und Förderglied 12 bezeichnet. Das Dosier- und Förderglied 12 ist zur Erfüllung der Dosierfunktion relativ zu dem Gehäuse 1 um die Achse A drehbeweglich, führt also bei der Einstellung der Dosis eine Dosierdrehbewegung um die Achse A aus. Zur Erfüllung der Förderfunktion ist es längs der Achse A in die Vortriebsrichtung translatorisch beweglich. Ferner bildet das Dosier- und Förderglied 12 mit einem proximalen Endabschnitt das Betätigungselement 13. Am Betätigungselement 13 nimmt der Patient die Einstellung der Dosis vor und bewirkt durch Ausübung einer Druckkraft in die Vortriebsrichtung auch die Ausschüttbewegung des Dosier- und Förderglieds 12. Zur Erfüllung der unterschiedlichen Funktionen, nämlich des Primens, des Dosierens und des Förderns bzw. Ausschüttens, weist das Dosier- und Förderglied 12 mehrere Strukturelemente auf, insbesondere ein erstes Dosierelement 14 für das Dosieren, das gleichzeitig auch einen Ausschüttanschlag bildet und somit selbst sowohl eine Dosierfunktion als auch eine Ausschüttfunktion erfüllt. Ein Dosierblockierelement 16 ist ein weiteres dieser Strukturelemente.

Das erste Dosierelement 14 wirkt bei dem Einstellen der Dosis mit mehreren zweiten Dosierelementen 24ᵢ zusammen, die um die Längsachse A verteilt angeordnet und in einem Dosierabschnitt 1a des Gehäuses 1 an dessen Mantelinnenfläche geformt sind. Die Dosierelemente 24ᵢ, mit i = 1, 2, 3......n, sind axiale Führungen für das erste Dosierelement 14, das mit diesen Führungen 24ᵢ als Eingriffselement zusammenwirkt. Die Dosierelemente bzw. Führungen 24ᵢ weisen unterschiedliche axiale Längen auf, wobei diese Längen jeweils mit einer einstellbaren Dosis korrespondieren. Die Dosierelemente 24ᵢ sind beispielhaft am Innenumfang des Dosierabschnitts 1a als axiale Sacknuten gebildet. Die Sacknuten sind an ihren proximalen Enden offen, so dass das erste Dosierelement 14 entsprechend der Drehwinkelposition des Dosier- und Förderglieds 12 in eine dieser Nuten 24ᵢ in die Vortriebsrichtung einfahren und im Verlaufe eines Ausschütthubs in der betreffenden Nut 24ᵢ bis zu deren distalen Ende in die Vortriebsrichtung bewegt werden kann. Das Dosierelement 14 bildet wie bereits erwähnt in Doppelfunktion einen Ausschüttanschlag, indem es am Ende des Ausschütthubs des Dosier- und Förderglieds 12 gegen einen Ausschüttanschlag 21 des Gehäuses 1, den im Beispiel das distale Ende der jeweiligen Sacknut 24ᵢ bildet, in axialen Anschlagkontakt gelangt. Die Länge des Ausschütthubs entspricht somit der Länge des in Abhängigkeit von der eingestellten Dosis mit dem Dosierelement 14 zusammenwirkenden Dosierelements 24ᵢ. Für die Dosierung kann alternativ ein Treppenglied vorgesehen sein, welches im Bereich des Dosierabschnitts 1a des Gehäuses 1 an dessen Mantelinnenfläche geformt ist. Das Treppenglied weist mehrere auf unterschiedlichen axialen Positionen angeordnete Axialanschläge auf. Für die Dosierung wird das erste Dosierelement 14 entsprechend der Drehwinkelposition des Dosier- und Förderglieds 12 in eine axiale Flucht mit einem der gewünschten Axialanschläge gebracht. Zwischen der zu verabreichenden Dosis und dem Abstand zwischen dem ersten Dosierelement 14 und dem durch die Drehwinkelposition ausgewählten Axialanschlag und der auszuschüttenden Dosis besteht ein Zusammenhang wie zwischen dem ersten Dosierelement 14 und dem distalen Ende der Sacknuten 24ᵢ. Die Verabreichung erfolgt wie bei der zuvor beschriebenen Alternative dadurch, dass das erste Dosierelement 14 bis an den zuvor ausgewählten Axialanschlag bewegt wird. Das Treppenglied ist insbesondere kostengünstig, da keine axialen Sacknuten benötigt werden.

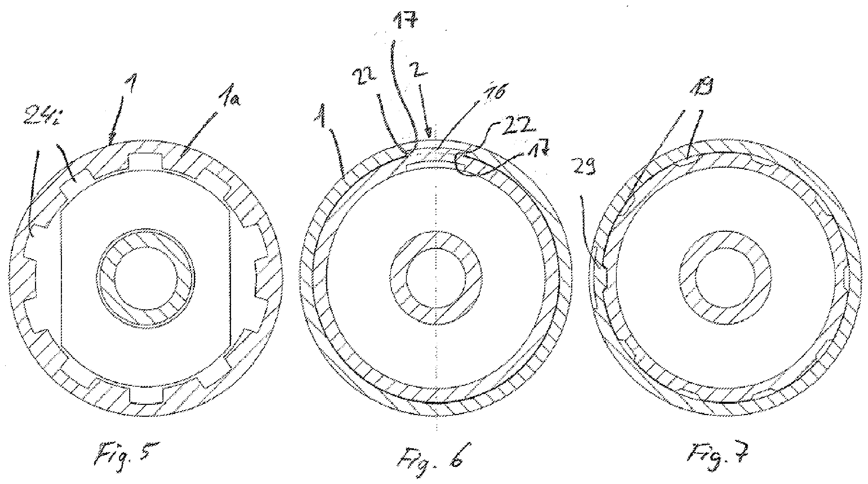

Das Dosier- und Förderglied 12 bildet mit dem Gehäuse 1 eine Dosierrasteinrichtung. An einem äußeren Umfang des Dosier- und Förderglieds 12 ist hierfür eine um die Rotationsachse A erstreckte Dosierraststruktur 19 geformt, wie am besten aus Figur 7 ersichtlich ist. Bei einer Drehbewegung des Dosier- und Förderglieds 12 gleitet ein an dem Gehäuse 1 geformtes, elastisch nachgiebiges Dosierrastelement 29 in Form eines radial nachgiebigen Schnappers über die Dosierraststruktur 19. Die Dosierraststruktur 19 ist in der Art einer Außenverzahnung gebildet, die beispielhaft unmittelbar an der Mantelaußenfläche des Dosier- und Förderglieds 12 vorzugsweise umlaufend um die Achse A geformt ist und deren Teilung mit der Teilung der zweiten Dosierelemente 24ᵢ korrespondiert, so dass das Dosierrastelement 29 jeweils in eine Vertiefung der Dosierraststruktur 19 eingreift, wenn das Dosierelement 14 gerade in axialer Flucht mit einem der Dosierelemente 24ᵢ ist. In Umkehrung der Verhältnisse könnte auch eine Dosierraststruktur an der Mantelinnenfläche des Gehäuses 1 und ein elastisch nachgiebiges Dosierrastelement am Dosier- und Förderglied 12 geformt sein.

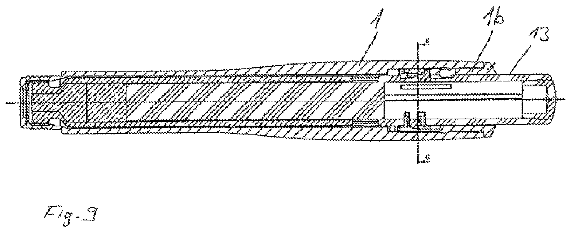

In Figur 9 wird ein modifiziertes Verabreichungsgerät gezeigt, das bis auf die im Folgenden erwähnten Unterschiede so aufgebaut ist bzw. mit den hierin beschriebenen Nadelschutzhülsen ausgestattet werden kann, wie in den Figuren 1 bis 8 beschrieben wird. Die Verabreichungsvorrichtung aus Figur 9 umfasst eine am proximalen Ende des Gehäuses 1 aufgenommene Funktionshülse 1b, die dreh- und axialfest mit dem Gehäuse insbesondere formschlüssig verbunden, wie z.B. verschnappt ist. Die Funktionshülse 1b weist einen Abschnitt auf, der von dem Gehäuse 1 umgeben wird. Ferner weist die Funktionshülse 1b einen Abschnitt auf, der das proximale Ende des Gehäuses bildet. Dieser Abschnitt weist eine Schulter auf, die am proximalen Ende des Gehäuses 1 anliegt und vorzugsweise auch in radialer Richtung bündig mit dem Gehäuse 1 abschließt. Die Funktionshülse 1b bringt Vorteile beim Montieren des Verabreichungsgeräts. Die Funktionshülse 1b kann die im hinteren Bereich des Injektionsgeräts aus Figur 1 gezeigten Einrichtungen aufweisen. Im Besonderen kann die Funktionshülse 1b einige oder die meisten Funktionen des Gehäuses 1 aus Figur 1 übernehmen, insbesondere eine Verdrehsicherung vor dem Primen oder/und eine Rasterung beim Einstellen der Dosierung oder/und eine axiale Führung der Kolbenstange.

Hierzu kann die Funktionshülse 1b das z. B. in Figur 7 gezeigte Dosierrastelement 29 aufweisen. Alternativ oder zusätzlich kann die Funktionshülse 1b das in Figur 6 gezeigte Dosisanzeigefenster 2 bilden, welches die Verdrehanschläge 22 bildet und in die das Dosierblockierelement 16 eingreift. Alternativ oder zusätzlich kann die Funktionshülse 1b den in Figur 5 gezeigten Dosierabschnitt 1a oder die ebenfalls in Figur 5 gezeigten Dosierelemente 24ᵢ aufweisen, um eine Längsführung des Dosier- und Förderglieds 12 zu bewirken.

Im Ausführungsbeispiel sind alle funktionalen Strukturelemente entweder am Gehäuse 1 oder am Dosier- und Förderglied 12 geformt. In einer ebenfalls bevorzugten Modifikation können das Dosierblockierelement 16 und die Dosierraststruktur 19 auch getrennt von dem Rest des Dosier- und Förderglieds 12 geformt werden, insbesondere an einem Hülsenteil entsprechend dem Hülsenteil des Ausführungsbeispiels, das den im vorstehenden Absatz genannten äußeren Umfang bildet. Solch ein getrennt geformtes Hülsenteil mit dem Dosierblockierelement 16 und der Dosierraststruktur 19 wäre allerdings bevorzugt unbeweglich mit dem das Gehäuse 1 durchragenden, die Kolbenstange bildenden Teil des Dosier- und Förderglieds 12 fest gefügt. Im Ergebnis würde ein gefügtes Dosier- und Förderglied 12 erhalten werden, das der Form nach dem des Ausführungsbeispiels entspräche.

Im Ausgangszustand des Verabreichungsgeräts (Figuren 1, 2a und 2b) befindet sich das Dosier- und Förderglied 12 in einem Dosierblockiereingriff, in dem es an einer Drehbewegung um die Achse A gehindert ist. Der Dosierblockiereingriff wird mittels des Dosierblockierelements 16 bewirkt, das ebenfalls an dem Dosier- und Förderglied 12 geformt ist, wie besonders gut in Figur 6 erkennbar ist. Das Dosierblockierelement 16 ist mit dem Gehäuse 1 in dem Dosierblockiereingriff. Das Gehäuse 1 bildet hiefür einen Verdrehanschlag 22 als Blockiergegenelement. Der Dosierblockiereingriff wird auf besonders einfache Weise dadurch gebildet, dass das Dosierblockierelement 16 im Ausgangszustand, in dem das Dosier- und Förderglied 12 seine in Bezug auf die Vortriebsrichtung hinterste Position relativ zum Gehäuse 1 und zum Reservoir 30 einnimmt, in das Dosisanzeigefenster 2 ragt, so dass dessen linke und rechte Seitenwand im und gegen den Uhrzeigersinn um die Achse A jeweils als ein Verdrehanschlag 22 wirken.

In Figur 6 ist der Bereich, in dem im Ausgangszustand des Geräts der Dosierblockiereingriff besteht, vergrößert im Detail dargestellt. Das Dosierblockierelement 16 ragt über eine äußere Umfangsfläche des Dosier- und Förderglieds 12 radial nach außen in das Dosisanzeigefenster 2 hinein. Es ist am Dosier- und Förderglied 12 so angeordnet, dass es in Bezug auf die Vortriebsrichtung unmittelbar hinter einer vorderen Stirnwand 23 des Fensters 2 in dieses hinein vorsteht und somit dieser Stirnwand 23 (Figur 2a) axial gegenüberliegt. Für den Dosierblockiereingriff, der im Ausführungsbeispiel aufgrund der Dosierdrehbeweglichkeit des Dosier- und Förderglieds 12 als Verdrehblockiereingriff gebildet ist, weist das Dosierblockierelement 16 in Umfangsrichtung um die Achse A, die Rotationsachse des Dosier- und Förderglieds 12, solch eine Breite auf, dass es wie in Figur 1 erkennbar das Fenster 2 in Umfangsrichtung nahezu vollständig ausfüllt, im Dosierblockiereingriff also in Umfangsrichtung keine "Luft" hat und quasi in enger Passung in beide Drehrichtungen den von den beiden Seitenwänden des Fensters 2 gebildeten Verdrehanschlägen 22 gegenüberliegt. Die in die beiden Drehrichtungen der Drehbeweglichkeit wirksamen Anschlagflächen des Dosierblockierelements 16 sind mit dem Bezugszeichen 17 versehen.

Das Dosierblockierelement 16 weist über die beiden Verdrehanschlagflächen 17 hinaus eine in Bezug auf die Achse A geneigte axiale Führungsfläche 18 auf, die mit der ihr axial gegenüberliegenden Stirnfläche 23 des Dosisanzeigefensters 2 zusammenwirkt. Wird auf das Dosier- und Förderglied 12 eine ausreichend große axiale Druckkraft ausgeübt, nämlich durch Druck auf das Bedienelement 13, wird das Dosierblockierglied 16 mit seiner geneigten Führungsfläche 18 axial gegen die Stirnfläche 23 gedrückt. Das Dosierblockierglied 16 ist in Bezug auf die Achse A radial elastisch nachgiebig, so dass es aufgrund der geneigten Führungsfläche 18 an der Stirnfläche 23 abgleitet und dabei nach radial innen und somit aus dem Dosierblockiereingriff mit dem in Bezug auf die Drehrichtung linken und rechten Verdrehanschlag 22 gelangt. Die Stirnfläche bildet einen Deblockieranschlag 23.

Wenn das Dosierblockierelement 16 gerade den Deblockieranschlag 23 in die Vortriebsrichtung passiert hat, ist das Dosier- und Förderglied 12 vom Dosierblockiereingriff frei und um die Achse A drehbeweglich.

Figur 5 zeigt das Verabreichungsgerät in einem Querschnitt im Bereich des Dosierabschnitts 1a während der Ausschüttung des Medikaments. Das Dosierelement 14 wird von einem der gleichmäßig um die Achse A im Dosierabschnitt 1a geformten Dosierelemente 24ᵢ axial geführt, so dass das Dosier- und Förderglied 12 während seines Ausschütthubs die bei der Dosisauswahl eingestellte Drehwinkelposition zwangsweise beibehält. Die Figuren 6 und 10 zeigen das Verabreichungsgerät nach vollständiger Ausschüttung der eingestellten Dosis, beispielhaft wurde die Maximaldosis ausgeschüttet.

Der Ausschütthub wird am in Vortriebsrichtung vorderen Ende jedes der Dosierelemente 24ᵢ durch den jeweiligen Ausschüttanschlag 21 beendet, indem das Dosierelement 14 gegen den jeweiligen Ausschüttanschlag 21 des Gehäuses 1 in die Vortriebsrichtung auf Anschlag gelangt. Jedes der Dosierelemente 24ᵢ weist an seinem in Vortriebsrichtung vorderen Ende einen Ausschüttanschlag 21 auf, wobei die Ausschüttanschläge 21 den unterschiedlichen Dosen entsprechend auf unterschiedlichen axialen Höhen geformt sind.

Das Verabreichungsgerät weist als weitere Besonderheit eine Rückhalteeinrichtung für das Dosier- und Förderglied 12 auf. Die Rückhalteeinrichtung sorgt dafür, dass das Dosier- und Förderglied 12 nach vollständiger Ausführung des Ausschütthubs in der dann eingenommenen Ausschüttposition verbleibt. Sie umfasst Rückhalteelemente 28, die den Dosierelementen 24ᵢ zugeordnet sind, nämlich jeweils eines der Rückhalteelemente 28 für jedes der Dosierelemente 24ᵢ. In Figur 6 sind das Rückhalteelement 28 des axial längsten Dosierelements 24ᵢ und das Rückhalteelement 28 des axial kürzesten Dosierelements 24ᵢ zu erkennen. Die Rückhalteelemente 28 werden vom Gehäuse 1 gebildet und sind beispielhaft an dessen Innenmantelfläche als nach radial innen vorragende Rückhaltenocken geformt. Der Ausschüttanschlag 14, respektive das Dosierelement 14, ist an einer in die Vortriebsrichtung weisenden Vorderseite in Bezug auf die Achse A geneigt, so dass er/es in die Vortriebsrichtung über das Rückhalteelement 28 des jeweiligen Dosierelements 24ᵢ gleiten kann. Die Rückseite des Ausschüttanschlags 14, respektive Dosierelements 14, weist zumindest im Wesentlichen orthogonal zur Achse A, so dass er/es im Zusammenwirken mit dem jeweiligen Rückhalteelement 28 das Dosier- und Förderglied 12 in dessen Ausschüttposition hält, entweder gegen eine Bewegung entgegen der Vortriebsrichtung blockiert oder eine derartige Rückziehbewegung zumindest behindert. Dies sorgt dafür, dass das Verabreichungsgerät nicht unbedacht zweckfremd für eine weitere Ausschüttung verwendet werden kann, sollte die eingestellte Dosis kleiner als die im Reservoir 30 im Ausgangszustand befindliche Medikamentendosis sein.

Nachfolgend wird die Funktionsweise des Verabreichungsgeräts im Zusammenhang erläutert:
Der Patient erhält das Gerät im Ausgangszustand der Figur 1. Zur Verabreichung entfernt er die Nadelschutzkappe 9 von dem als Spritze ausgestalteten Reservoir 30.

Vor der Verabreichung muss das Reservoir 30 entlüftet werden. Das Verabreichungsgerät erzwingt diesen Primingschritt, da erst nach dem Primen die zu verabreichende Dosis eingestellt werden kann. Das beim Primen ausgestoßene Volumen ist vorgegeben, nämlich durch den axialen lichten Abstand, den der Ausschüttanschlag 14 vom Priminganschlag 25 aufweist. Ein vorzeitiges Einstellen der Dosis wird durch den Dosierblockiereingriff zwischen dem Dosierblockierelement 16 und dem Verdrehanschlag 22 des Gehäuses 1 verhindert. Der Dosierblockiereingriff zwischen 16 und 21 ist insbesondere auch in Figur 1 erkennbar.

In diesem Ausgangszustand liefert die Dosisanzeige dem Patienten den Hinweis, dass er ein Primen ausführen muss und wie dies geschieht. Im Dosisanzeigefenster 2 erscheint die in Figur 1 erkennbare Anzeige, ein Piktogramm, eines in die Vortriebsrichtung weisenden Richtungspfeils oder/und eines "P" für "Primen". Der Patient entlüftet das Reservoir 30 durch Ausübung einer axialen Druckkraft auf das Bedienelement 13. Unter der Druckkraft gibt das elastisch nachgiebige Dosierblockierelement 16 radial nach innen nach, indem seine Führungsfläche 18 über den Deblockieranschlag 23 des Gehäuses 1 gleitet und das Dosierblockierelement 16 durch dieses Abgleiten nach radial innen aus dem Dosierblockiereingriff bewegt wird. Der Priminghub wird durch den Priminganschlag 25 begrenzt, d. h. der Priminghub ist beendet, sobald der Ausschüttanschlag 14 gegen den Priminganschlag 25 auf Anschlag gelangt.

Unmittelbar nach Ausführung des Priminghubs ist der Dosierblockiereingriff gelöst. In diesem Zustand kann durch Drehen des Dosier- und Förderglieds 12 um die Achse A die zu verabreichende Dosis eingestellt werden, indem das Dosier- und Förderglied 12 um die Achse A in eine Drehwinkelposition gedreht wird, in der der Ausschüttanschlag 14, der beim Dosieren als Dosierelement 14 wirkt, in axialer Flucht mit demjenigen der Dosierelemente 24ᵢ ist, das seiner axialen Länge nach der zu verabreichenden Dosis entspricht. Im Dosisanzeigefenster 2 ist die eingestellte Dosis ablesbar (Figur 2).

Nach dem Einstellen der Dosis drückt der Patient das Verabreichungsgerät mit dem distalen Ende der Nadelschutzhülse 5 an die gewünschte Einstechstelle, wodurch sich die Nadelschutzhülse 5 aus ihrer ersten ausgefahenen Position in die eingefahrene Position bewegt und die Injektionsnadel 7 in und unter die Haut sticht. Nach dem Einstechen wird die eingestellte Dosis ausgeschüttet, indem der Patient durch Ausübung einer axialen Druckkraft auf das Bedienelement 13 das Dosier- und Förderglied 12 in die Vortriebsrichtung bewegt. Das Dosier- und Förderglied 12 drückt dabei gegen den Kolben 11 und diesen im Reservoir 30 in Richtung auf den Auslass vor, bis das Dosier- und Förderglied 12 mit seinem Ausschüttanschlag 14 gegen den Ausschüttanschlag 21 stößt, der dem ausgewählten Dosierelement 24ᵢ zugeordnet ist. Der Ausschütthub des Dosier- und Förderglieds 12 und des Kolbens 11, also der Fördereinrichtung 10, entspricht der axialen Länge des ausgewählten Dosierelements 24ᵢ.

Kurz vor dem Ende des Ausschütthubs überfährt der Ausschüttanschlag 14 (Dosierelement 14) das dem ausgewählten Dosierelement 24ᵢ zugeordnete Rückhalteelement 28. Das hierbei entstehende Klick-Geräusch vermittelt dem Patienten ein hörbares und auch fühlbares Signal dafür, dass der Ausschütthub vollständig ausgeführt wurde. Das Dosier- und Förderglied 12 befindet sich nun im Rückhalteeingriff, der ein Zurückziehen des Dosier- und Förderglieds 12 gegen die Vortriebsrichtung verhindert oder zumindest stark behindert. Wie in Figur 4a erkennbar, liefert die Dosisanzeige durch das Fenster 2 hindurch eine Blindanzeige. In einer Weiterentwicklung kann die Dosisanzeige 15 um ein Piktogramm oder eine Farbmarkierung oder auch einen Text ergänzt werden, um dem Patienten eine positive Rückmeldung des Inhalts zu liefern, dass die eingestellte Dosis vollständig ausgeschüttet wurde. Durch das Reservoirfenster 3 kann der Patient den Kolben 11 erkennen. Im Ergebnis wird ihm somit auch visuell signalisiert, dass die Ausschüttung vollständig durchgeführt wurde.

### Bezugszeichen:

- 1: Gehäuse
- 1a: Dosierabschnitt
- 1b: Funktionshülse
- 2: Dosisanzeigefenster
- 3: Reservoirfenster
- 4: Führungsbahn
- 4a-4c: Anschläge
- 4d: Flanke/Führungsfläche
- 4e: Umschaltelement
- 4f: Flanke/Führungsfläche
- 4g/4h: Anschläge
- 5: Nadelschutzhülse
- 5 a: Eingriffsglied
- 6: Federelement, Feder
- 6a: Eingriffsmittel
- 7: Injektionsnadel
- 8: Nadelschutzkappe
- 9: Federkulisse
- 9a: Führungsfläche
- 10: Fördereinrichtung
- 11: Förderglied, Kolben
- 12: Dosier- und Förderglied, Dosierglied, Förderglied
- 13: Bedienelement
- 14: Dosierelement, Ausschüttanschlag
- 15: Dosisskala
- 16: Dosierblockierelement
- 17: Verdrehgegenanschlag
- 18: Führungsfläche
- 19: Dosierraststruktur
- 20: -
- 21: Ausschüttanschlag
- 22: Verdrehanschlag
- 23: Deblockieranschlag
- 24ᵢ: Dosierelement, Führung
- 25: Priminganschlag
- 26: -
- 27: -
- 28: Rückhalteelement
- 29: Dosierrastelement
- 30: Reservoir, Spritze
- 31: Flansch
- 32: -

- A: Vortriebsachse, Rotationsachse

## Patentansprüche

1. Vorrichtung zum Schutz vor Verletzungen an einer Injektionsnadel, die Vorrichtung umfassend:
a) ein Gehäuse, (I),
b) eine Nadel (7), mit der ein flüssiges Produkt injizierbar ist,
c) eine Nadelschutzhülse (5), welche aus einer ausgefahrenen Position entgegen der Kraft einer Feder (6) in eine eingefahrene Position oder/und aus einer eingefahrenen Position durch die Kraft der Feder (6) in eine ausgefahrene Position bewegbar ist, wobei die Feder (6) einteilig an der Nadelschutzhülse (5) gebildet ist,
d) wobei die Feder (6) ein Federarm ist, der ein Eingriffsmittel (6a) aufweiset, das an eine quer zu der Nadellängsachse geneigte Führungsfläche (9a) eingreift, **dadurch gekennzeichnet, dass**
e) eine Ausnehmung (9) in dem Gehäuse (1) gebildet ist,
f) und die Führungsfläche (9a) eine Flanke der von dem Gehäuse (1) gebildeten Ausnehmung (9) ist.

2. Vorrichtung nach dem vorhergehenden Anspruch, **dadurch gekennzeichnet, dass** die Feder (6) eine Druckfeder ist, die sich am Gehäuse (1) abstützt.

3. Vorrichtung nach einem der vorhergehenden Ansprüche, **dadurch gekennzeichnet, dass** das Eingriffsmittel (6a) gegen die Führungsfläche (9a) drückt und die durch die Feder (6) auf die Nadelschutzhülse (5) aufgebrachte Kraft ein von der Neigung der Führungsfläche (9a) abhängiger und in Verschicherichtung der Nadelschutzhülse (5) weisender Kraftanteil ist.

4. Vorrichtung nach dem vorhergehenden Anspruch, **dadurch gekennzeichnet, dass** die von der Feder (6) unmittelbar ausgeübte Kraft über den Eingriff des Eingriffsmittels (6a) in die Führungsfläche (9a) in eine auf die Nadelschutzhülse (5) wirkende Axialkraft umwandelbar ist

5. Vorrichtung nach dem vorhergehenden Anspruch, **dadurch gekennzeichnet, dass** die unmittelbare Federkraft quer auf die Bewegungsrichtung der Nadelschutzhülse (5) steht.

6. Vorrichtung nach einem der vorhergehenden Ansprüche, **dadurch gekennzeichnet, dass** die Nadelschutzhülse (5) ein Eingriffsglieds (5a) aufweist, das in einer Ausgangsposition der Nadelschutzhülse (5) in einen Eingriff mit einem in distale Richtung wirkenden Anschlag (4c) ist, der eine Bewegung der Nadelschutzhülse (5) in distale Richtung sperrt und insbesondere einen Endanschlag bildet.

7. Vorrichtung nach dem vorhergehenden Anspruch, **dadurch gekennzeichnet, dass** das Eingriffsglied (5a) federnd angeordnet ist, wobei das Eingriffsglied (5a) bei einer Bewegung der Nadelschutzhülse (5) in proximale Richtung von einer Führungsfläche (4d) auslenkbar ist, wobei das Eingriffsglied (5a) bei der im Bezug auf die Ausgangsposition in proximale Richtung bewegten Position der Nadelschutzhülse (5) aus der ausgelenkten Position zurückfedert.

8. Vorrichtung nach dem vorhergehenden Anspruch **dadurch gekennzeichnet, dass** das Eingriffsglied (5a) bei einer Bewegung der Nadelschutzhülse (5) aus ihrer eingefahrenen Position in distale Richtung von einer Führungsfläche (4f) auslenkbar ist, wobei das Eingriffsglied (5a) in der ausgefahrenen Position der Nadelschutzhülse (5) aus der ausgelenkten Position zurückfedert.

9. Vorrichtung nach dem vorhergehenden Anspruch, **dadurch gekennzeichnet, dass** das Eingriffsglied (5a) bei aus der eingefahrenen Position in die ausgefahrene Position bewegter Nadelschutzhülse (5) zwischen einem in distale Richtung wirkenden Anschlag (4g), der eine Bewegung der Nadelschutzhülse (5) in distale Richtung sperrt, und einem in proximale Richtung wirkenden Anschlag (4h). der eine Bewegung der Nadelschutzhülse (5) in proximale Richtung sperrt, angeordnet ist.

10. Vorrichtung nach dem vorhergehenden Anspruch, **dadurch gekennzeichnet, dass** wenigstens einer der Anschläge (4c, 4g, 4h) und/oder wenigstens eine der Führungsflächen (4d, 4f) von einer Führungsbahn (4) gebildet ist, wobei die Führungsbahn (4) bevorzugt von dem Gehäuses (1) gebildet ist und in die Führungsbahn (4) das vorzugsweise nockenförmige Eingriffsglied (5a), insbesondere während sämtliche Verschiebebewegungen der Nadelschutzhülse (5), eingreift.

11. Vorrichtung nach einem der vorhergehenden Ansprüche, **dadurch gekennzeichnet, dass** das Gehäuse (1) ein Gehäuse einer Injektionsvorrichtung ist oder mit dem Gehäuse einer Injektionsvorrichtung verbindbar ist.

12. Injektionsvorrichtung umfassend eine Vorrichtung nach einem der vorhergehenden Ansprüche, ferner umfassend:
a) ein von dem Gehäuse (1) gebildetes oder aufgefangenes Produktbehältnis (30) oder Reservoir, vorzugsweise eine Karpule oder Spritze, in dem ein flüssiges Medikaments, vorzugsweise auf der Basis von FSH oder einer FSH-Variante enthalten ist,
b) und eine Fördereinrichtung (10) mit einem Förderglied zum Ausschütten des Medikaments,
c) wobei das Medikament nicht konserviert und
d) das Produktbehältnis (30) oder Reservoir steril geschlossen ist.

13. Injektionsvorrichtung nach dem vorhergehenden Anspruch, **dadurch gekennzeichnet, dass** das Produktbehältnis (30) im steril geschlossenen Zustand das Medikament in einer Menge enthalt, die höchstens so gross wie eine Maximaldosis ist in der das Medikament in einer einzigen Injektion verabreicht wird.

14. Injektionsvorrichtung nach einem der Ansprüche 12-13, ferner umfassend:
a) ein Dosierglied, (12-19), das zum Einstellen einer zu verabreichenden Dosis des Medikaments relativ zum Gehäuse (I) eine Dosierbewegung ausführen kann,
b) wobei die Fördereinrichtung (10) mit dem Förderglied (12-14) zum Ausschütten der eingestellten Dosis dient,
c) wobei das Dosierglied (12-19) in einem Ausgangszustand der Injektionsvorrichtung in einen lösbaren Dosierblockiereingriff (16, 22) in Richtung der Dosierbewegung blockiert,
d) und am Ende einer der Entlüftung des Produktbehältnisses (30) oder Reservoirs dienenden Primingbewegung des Förderglieds (12-14) von der Blockierung frei ist

15. Injektionsvorrichtung nach einem der Ansprüche 12-14
mit wenigstens einem der folgenden, insbesondere nicht konservierte Medikamente oder Wirkstoffe:
- Medikament auf der Basis von FSH oder einer FSH-Variante,
- Neuroleptikum,
- gefässerweiternden Mittel,
- Blutprodukt,
- Medikamente zur Behandlung einer rheumatischen Erkrankung,
- Onkologikum,
- Medikament zur Behandlung einer Infektionskrankheit.

## Claims

1. A device for protecting from injury on an injection needle, the device including:
a) a housing (1),
b) a needle (7) with which a liquid product can be injected,
c) a needle protection sleeve (5) moveable from an extended position against the force of a spring (6) into a retracted position and/or from a retracted position by the force of the spring (6) into an extended position, the spring (6) being formed in one piece on the needle protection sleeve (5),
d) wherein the spring (6) is a spring arm having an engagement means (6a) engaging a guide surface (9a) which is inclined transversely relative to the longitudinal axis of the needle,
**characterised in that**
e) a recess (9) is formed in the housing (1), and
f) the guide surface (9a) is a flank of the recess (9) formed by the housing (1).

2. A device according to the preceding claim **characterised in that** the spring (6) is a compression spring which is supported against the housing (1).

3. A device according to one of the preceding claims **characterised in that** the engagement means (6a) presses against the guide surface (9a) and the force applied to the needle protection sleeve (5) by the spring (6) is a force component which is dependent on the inclination of the guide surface (9a) and which faces in the direction of displacement of the needle protection sleeve (5).

4. A device according to the preceding claim **characterised in that** the force exerted directly by the spring (6) can be converted by way of the engagement of the engagement means (6a) into the guide surface (9a) into an axial force acting on the needle protection sleeve (5).

5. A device according to the preceding claims **characterised in that** the direct spring force is transverse relative to the direction of movement of the needle protection sleeve (5).

6. A device according to one of the preceding claims **characterised in that** the needle protection sleeve (5) has an injection member (5a) which in a starting position of the needle protection sleeve (5) is in a condition of engagement with an abutment (4c) which acts in the distal direction and which blocks a movement of the needle protection sleeve (5) in the distal direction and in particular forms an end abutment.

7. A device according to the preceding claim **characterised in that** the engagement member (5a) is arranged resiliently, wherein the engagement member (5a) can be deflected by a guide surface (4d) upon a movement of the needle protection sleeve (5) in the proximal direction, wherein the engagement member (5a) springs back out of the deflected position in the position of the needle protection sleeve (5) of being moved in the proximal direction in relation to the starting position.

8. A device according to the preceding claim **characterised in that** the engagement member (5a) can be deflected by a guide surface (4f) upon a movement of the needle protection sleeve (5) out of its retracted position in the distal direction, wherein the engagement member (5a) springs back out of the deflected position in the extended position of the needle protection sleeve (5).

9. A device according to the preceding claim **characterised in that** with the needle protection sleeve (5) moved out of the retracted position into the extended position the engagement member (5a) is arranged between an abutment (4g) which is operative in the distal direction and which blocks a movement of the needle protection sleeve (5) in the distal direction and an abutment (4h) which is operative in the proximal direction and which blocks a movement of the needle protection sleeve (5) in the proximal direction.

10. A device according to the preceding claim **characterised in that** at least one of the abutments (4c, 4g, 4h) and/or at least one of the guide surfaces (4d, 4f) is formed by a guide track (4), wherein the guide track (4) is preferably formed by the housing (1) and the preferably cam-shaped engagement member (5a) engages into the guide track (4), in particular during all displacement movements of the needle protection sleeve (5).

11. A device according to one of the preceding claims **characterised in that** the housing (1) is a housing of an injection device or can be connected to the housing of an injection device.

12. An injection device including a device according to one of the preceding claims and further including:
a) a product container (30) or reservoir formed or held by the housing (1), preferably a cartridge or syringe, in which a liquid drug, preferably on the basis of FSH or an FSH variant, is contained, and
b) a conveyor device (10) having a conveyor member for dispensing the drug,
c) wherein the drug is not preserved, and
d) the product container (30) or reservoir is sterilely closed.

13. An injection device according to the preceding claim **characterised in that** the product container (30) in the sterilely closed state contains the drug in an amount which at most is as great as a maximum dose in which the drug is administered in a single injection.

14. An injection device according to one of claims 12 and 13 and further including:
a) a dosing member (12-19) which can perform a dosing movement relative to the housing (1) to set a dose to be administered of the drug,
b) wherein the conveyor device (10) with the conveyor member (12-14) serves to dispense the set dose,
c) wherein in a starting condition of the injection device the dosing member (12-19) is blocked in the direction of the dosing movement in a releasable dosing blocking engagement (16, 22), and
d) is free of the block at the end of a priming movement of the conveyor member (12-14) that serves to vent the product container (30) or reservoir.

15. An injection device according to one of claims 12 to 14 comprising at least one of the following, in particular non-preserved drugs or active agents:
- a drug on the basis of FSH or an FSH variant,
- a neuroleptic drug,
- a vasodilative agent,
- blood product,
- a drug for treating a rheumatic disease,
- an oncological drug, and
- a drug for treating an infectious disease.

## Revendications

1. Dispositif pour la protection contre des blessures avec une aiguille d'injection, le dispositif comprenant :
a) un logement (1),
b) une aiguille (7) avec laquelle un produit liquide peut être injecté,
c) une gaine de protection d'aiguille (5) qui peut être déplacée d'une position déployée à l'encontre de la force d'un ressort (6) à une position rentrée et/ou d'une position rentrée par la force du ressort (6) à une position déployée, dans lequel le ressort (6) est formé d'un seul tenant au niveau de la gaine de protection d'aiguille (5),
d) dans lequel le ressort (6) est un bras de ressort qui présente un moyen de mise en prise (6a) qui met en prise une surface de guidage (9a) inclinée perpendiculairement à l'axe longitudinal de l'aiguille,
**caractérisé en ce que**
e) un évidement (9) est formé dans le logement (1),
f) et la surface de guidage (9a) est un flanc de l'évidement (9) formé par le logement (1).

2. Dispositif selon la revendication précédente, **caractérisé en ce que** le ressort (6) est un ressort à compression qui s'appuie sur le logement (1).

3. Dispositif selon l'une des revendications précédentes, **caractérisé en ce que** le moyen de mise en prise (6a) appuie contre la surface de guidage (9a) et la force appliquée par le ressort (6) sur la gaine de protection d'aiguille (5) est une proportion de force qui dépend de l'inclinaison de la surface de guidage (9a) et qui est orientée dans la direction de décalage de la gaine de protection d'aiguille (5).

4. Dispositif selon la revendication précédente, **caractérisé en ce que** la force exercée directement par le ressort (6) peut être convertie par la mise en prise du moyen de mise en prise (6a) dans la surface de guidage (9a) en une force axiale agissant sur la gaine de protection d'aiguille (5).

5. Dispositif selon la revendication précédente, **caractérisé en ce que** la force de ressort directe est perpendiculaire à la direction de mouvement de la gaine de protection d'aiguille (5).

6. Dispositif selon l'une des revendications précédentes, **caractérisé en ce que** la gaine de protection d'aiguille (5) présente un membre de mise en prise (5a) qui, dans une position de départ de la gaine de protection d'aiguille (5), est en prise avec une butée (4c) agissant dans une direction distale qui bloque un mouvement de la gaine de protection d'aiguille (5) dans une direction distale et forme en particulier une butée terminale.

7. Dispositif selon la revendication précédente, **caractérisé en ce que** le membre de mise en prise (5a) est disposé de façon élastique, dans lequel le membre de mise en prise (5a) peut être dévié par un mouvement de la gaine de protection d'aiguille (5) en direction proximale d'une surface de guidage (4d), dans lequel le membre de mise en prise (5a) a un rappel élastique, dans la position déplacée en direction proximale par rapport à la position de départ de la gaine de protection d'aiguille (5), à partir de la position déviée.

8. Dispositif selon la revendication précédente, **caractérisé en ce que** l'élément de mise en prise (5a) peut être dévié lors d'un mouvement de la gaine de protection d'aiguille (5) à partir de sa position rentrée dans la direction distale d'une surface de guidage (4f), dans lequel le membre de mise en prise (5a) a un rappel élastique, dans la position déployée de la gaine de protection d'aiguille (5), à partir de la position déviée.

9. Dispositif selon la revendication précédente, **caractérisé en ce que** le membre de mise en prise (5a) est disposé, lorsque la gaine de protection d'aiguille (5) est déplacée de la position rentrée à la position déployée, entre une butée (4g) agissant en direction distale, qui bloque un mouvement de la gaine de protection d'aiguille (5) en direction distale, et une butée (4h) agissant dans une direction proximale, qui bloque un mouvement de la gaine de protection d'aiguille (5) en direction proximale.

10. Dispositif selon la revendication précédente, **caractérisé en ce qu'**au moins l'une des butées (4c, 4g, 4h) et/ou au moins l'une des surfaces de guidage (4d, 4f) est formé par une voie de guidage (4), dans lequel la voie de guidage (4) est formée de préférence par le logement (1) et dans la voie de guidage (4) le membre de mise en prise (5a) de préférence en forme de came est en prise, en particulier pendant tous les mouvements de décalage de la gaine de protection d'aiguille (5).

11. Dispositif selon l'une des revendications précédentes, **caractérisé en ce que** le logement (1) est un logement d'un dispositif d'injection ou peut être relié au logement d'un dispositif d'injection.

12. Dispositif d'injection comprenant un dispositif selon l'une des revendications précédentes, comprenant en outre :
a) un conteneur de produit (30) formé ou reçu par le logement (1) ou un réservoir, de préférence une cartouche ou une seringue, dans lequel un médicament liquide, de préférence à base de FSH ou une variante de FSH, est contenu,
b) et un système de transport (10) avec un élément de transport pour distribuer le médicament,
c) dans lequel le médicament n'est pas conservé et
d) le conteneur de produit (30) ou le réservoir est fermé de façon stérile.

13. Dispositif d'injection selon la revendication précédente, **caractérisé en ce que** le conteneur de produit (30) à l'état stérile fermé contient le médicament en une quantité qui est au plus aussi importante que la dose maximale dans laquelle le médicament peut être administré en une injection unique.

14. Dispositif d'injection selon l'une des revendications 12 à 13, comprenant en outre :
a) un élément de dosage (12-19) qui peut exécuter un mouvement de dosage pour ajuster une dose à administrer du médicament par rapport au logement (1),
b) dans lequel le système de transport (10) avec l'élément de transport (12-14) sert à distribuer la dose ajustée,
c) dans lequel l'élément de dosage (12-19) bloque dans un état de départ du dispositif d'injection dans une mise en prise de blocage de dosage détachable (16, 22) dans la direction du mouvement de dosage,
d) et à la fin d'un mouvement d'amorçage de l'élément de transport (12-14) servant à la ventilation du conteneur de produit (30) ou du réservoir, est libéré du blocage.

15. Dispositif d'injection selon l'une des revendications 12 à 14, avec au moins l'un des médicaments ou principes actifs suivants, en particulier non conservés :
- médicament à base de FSH ou d'une variante de FSH,
- neuroleptique,
- agent vasodilatateur,
- produit sanguin
- médicament pour le traitement d'une maladie rhumatismale,
- agent antinéoplasique
- médicament pour le traitement d'une maladie infectieuse.
